# EUROPEAN PATENT APPLICATION

(11) **EP 1 308 516 A1**
(43) Date of publication of application: **07.05.2003**
(21) Application number: 01402754.4
(22) Date of filing: 24.10.2001
(51) Int. Cl.: C12N 15/85, C12N 15/62, C12N 15/90, C12N 15/63

(54) **Site-directed recombinase fusion proteins and corresponding polynucleotides, vectors and kits, and their uses for site-directed DNA recombination**

(71) Applicant: ADEREGEM (Association pour le Développement de la Recherche en Génétique Moléculaire, F-75012 Paris (FR)
(72) Inventor: Mueller, Ferenc, 76021 Karlsruhe (DE); Straehle, Uwe, 77694 Kehl (DE); Tora, Laszlo, 67880 Krautergersheim (FR); Olasz, Ferenc, 2100 Godollo (HU); Kiss, Janos, 2100 Godollo (HU); Szabo, Monika, 2143 Kistarcsa (HU)
(74) Representative: Ahner, Francis

(57) **Abstract**

The invention relates to fusion proteins comprising a site-directed recombinase protein and a heterologous protein that binds either directly or indirectly DNA at at least one responsive element, said fusion protein having at least a heterologous site-directed recombinase activity. The invention also relates to the corresponding polynucleotides, vector and kits. The invention also provides methods for site-directed DNA recombination and for the stable introduction a DNA sequence of interest into a recipient DNA molecule.

## Description

This invention relates to the field of biology, more particularly to the field of recombinant DNA technology. The invention relates to novel recombinant recombinase proteins and their use to catalyse DNA site-specific recombination. The invention also relates to methods for site-specific recombination *in vitro* or *in vivo,* as well as polynucleotides and vectors which can be employed in such methods. The proteins, vectors and methods of the present invention can be used to stably integrate a DNA sequence of interest into a recipient DNA.

Manipulations of gene expression involve the introduction of transfected genes (transgenes) to confer some novel property upon, or to alter some intrinsic property, of cells or organisms. The efficacy of such manipulations is often impaired by such problems as the inability to control chromosomal site of transgene integration, or the inability to control the number of copies of a transgene that integrate at the desired chromosomal site, or by the difficulties in controlling the level, temporal characteristics, or tissue distribution of transgene expression, or by the difficulty of modifying the structure of transgenes once they are integrated into mammalian chromosomes. Thus the basic problems are inability to insert and retrieve any DNA segment with precision, good yields, efficiency and facility.

Site-specific recombination systems constitute means to circumvent the above mentioned problems. So far mainly two systems are routinely used to perform site-specific recombination in DNA ; there may be mentioned the Cre/Lox (Sauer, 1998) and FLP/FRT (Kilby *et al.,* 1993) systems. These systems are based on the use of recombinases which catalyze the recombination reaction between two short recognition DNA sequences. It has been shown that these site-specific recombination systems, although of microbial origin for the majority, could function in higher eukaryotes, such as plants, insects and mice (Sauer, 1994; Rajewsky *et al*., 1996; Sauer, 1998).

Additional site-specific recombination systems based on transposons have been developed such as the site-specific recombination system of HP1 bacteriophage, the site-specific recombination system used by the *Streptomyces* bacteriophage phi C31, the Tn3 site-specific recombination system (Stark *et al*., 1989), the V(D)J recombination system (Kim *et al.,* 2000), the integrons recombination system (Ploy *et al.,* 2000).

Beside the prior art recombination systems, there remains a need for an additional improved site-specific recombination system to allow the targeting of additional sites. Indeed existing site-specific recombination systems are limited to target sequences which contain the recognition site of the particular recombination system. In the system described in the invention the recognition site can be modified or exchanged with new targeting sites. Such additional site-specific recombinations systems would allow the simultaneous manipulation of transgenes in a host cell. The problem to be solved is to develop new site-specific recombinases with new targeting site(s).

The generally employed site-specific recombination techniques utilize the DNA binding characteristics of the given recombinase used. In accordance with the present invention, the inventors propose to add new DNA-binding specificity to transposases and recombinases by adding/replacing their DNA binding domains with that of heterologous proteins. An advantage of the system of the invention is that the multiplicity of recombinase and transposase genes available in the nature allow to create a multiplicity of chimeric recombinant site-specific recombinases that catalyse recombination at different site-directed recombinase targeting sequences (SDRTS). Moreover, another advantage of the present invention-based recombination technology is, that unlike the "cut and paste" type transposons, there is no size restriction of the DNA used as donor, allowing large DNA constructs (large plasmids, cosmids, PACs) to be integrated into target genomes.

The invention provides a fusion protein comprising at least (i) a site-directed recombinase protein, or a fragment or a variant thereof and, (ii) a heterologous protein, or a fragment or a variant thereof, that binds, either directly or indirectly, DNA at least at one responsive element (RE), said fusion protein having a heterologous site-directed recombinase activity such that in a cell or a cell free system containing said responsive element, said fusion protein binds directly or indirectly to said responsive element and catalyzes recombination in the vicinity or at said DNA responsive element in presence of site-directed recombinase targeting sequence (SDRTS). In a preferred embodiment, only a fragment corresponding to a functional DNA binding domain of the heterologous protein is fused to the site-directed recombinase protein, or fragment or a variant thereof. In a second preferred embodiment, only a fragment corresponding to the functional site-directed recombinase domain of said site-directed recombinase is fused to the heterologous protein, or a fragment or a variant thereof.

"Heterologous protein" refers to protein, which is preferably different from the protein used to bring the site-specific recombinase activity to the fusion protein of the invention.

Preferably, the endogenous DNA-binding domain of said site-directed recombinase protein or a fragment or a variant thereof, is no more functional in the fusion protein such that the fusion protein of the invention does not anymore catalyze recombination at the natural endogenous targeting sequence of said site-directed recombinase protein. The non-functional DNA binding domain of said endogenous site-directed recombinase has been rendered non-functional, either by partial or total deletion of said endogenous DNA-binding domain, either by mutation of said endogenous DNA-binding domain, or by the fusion to the heterologous DNA-binding domain.

Alternatively, it could be of interest that the fusion protein of the invention retains totally or partially its ability to bind DNA at its endogenous DNA-binding domain and to catalyse both recombination at the natural endogenous targeting sequence of said site-directed recombinase protein and at the heterologous targeting sequence.

The expression "recombinase fragment" is understood to mean any recombinase portion exhibiting at least one recombinase activity. The expression "heterologous protein fragment" means any portion of the heterologous protein exhibiting at least a site-specific DNA-binding activity either directly or indirectly.

A heterologous protein, or a fragment or a variant thereof that binds directly to DNA means that this protein is directly in contact with the double helix by recognizing a DNA sequence motif or a DNA conformation. Such binding between the protein and the DNA sequence is performed by the means of a series of weak bindings between the protein amino acids and the DNA bases. Such protein comprises conserved consensus regions easily recognizable by a man skilled in the art. Among those consensus regions, one can recite for example the zinc-finger motif, the leucine zipper motif, the helix-turn-helix motif.

A heterologous protein, or a fragment or a variant thereof that binds indirectly to DNA means that this protein binds to DNA through at least one additional protein. This additional protein comprises a DNA-binding domain and a protein-binding domain to interact with the heterologous protein of the invention.

The recombinase activity of the fusion protein of the invention, or fragments thereof, can be evaluated by the estimation of the frequency of recombination events catalyzed by said recombinase. This is estimated by techniques known to persons skilled in the art. These events may be revealed by PCR or Southern Blotting; the recombination frequency being estimated by taking the ratio of the representation of the various alleles in the cells of a tissue. The frequencies of the various alleles may be estimated by assaying the intensity of the corresponding bands on an electrophoresis gel of a product of PCR amplification or of genomic DNA (Southern blotting). The use of the PCR makes this method of estimation extremely sensitive and makes it possible to detect the presence of cells of the organism whose genome has not undergone targeted site-specific recombination. Another way of estimating the efficiency of the recombination may be carried out indirectly by immunohistochemistry, by analyzing for example the expression of the gene sequence that have been inactivated by the site-specific recombination event.

By "fragments of the fusion protein of the invention", it is meant any biologically active part of the fusion protein able to catalyze at least site-directed DNA recombination at the heterologous targeting sequence.

The site-specific DNA binding activity of said heterologous protein, or fragment thereof can be evaluated by techniques commonly used by the man skilled in the art, such as for examples, footprinting assay, gel shift assay, southwestern blotting.

The site-directed recombinase part of the fusion protein of the invention is selected among prokaryotic site-directed recombinase and eukaryotic site-directed recombinase. More preferably, said site-directed recombinase is selected in the group comprising transposase and DNA recombinase.

By "transposase", it is meant a protein, which serves to insert transposable elements into the genome of the recipient organism. The transposase protein recognizes specific DNA sequences (*e.g.* inverted repeat ends, SDRTS) and can catalyse the recombination reaction by joining these specific SDRTS sequences to target DNA. Target DNA can be a random DNA sequence or a more or less specific target site (*e.g*. transposition hot spot). Transposases are members of a class of enzymes which are termed *"cis-acting", i.e*., they function optimally to transpose DNA sequences located on the same molecule as the transposase gene(s) itself.

By "recombinase", it is meant a protein, which catalyses rearrangement of DNA sequences. There can be recombinases that catalyse homologous recombination (*e.g.* RecABC system of *E. coli)* or site specific recombination (*e.g.* att/int system of lambda bacteriophage ; Tn3 resolvase family of bacteria ; Cre recombinase of P1 bacteriophage, FLP recombinase of *saccharomyces ;* V(D)J recombinase RAG3 of the immune system, etc.).

Any transposase is in the scope of the present invention. In a preferred embodiment, said transposase is selected from the transposases encoded by sequences derived from a transposable element selected from the group consisting of:
- transposons Tn3, Tn5, Tn7, Tn10, Tn916; and
- procaryotic mobile elements IS1, IS2, IS3, IS5, IS10L, IS10L/R-2, IS26, IS30, IS50, IS150, IS186, IS911, and
- eukaryotic mobile elements Tc1 of *Caenorhabditis elegans,* the P and *Copia* elements of *Drosophila*, the synthetic element Sleeping Beauty, Hobo, Mariner, Ac-Ds Spm, the Mu elements of maize, Ty1, Ty2, Ty3 elements of yeast, Tam1, Tam2 and Tam3 elements of *Antirrhinum,* Tx1 and Tx2 elements of *Xenopus* ; and
- retrotransposons such as blood, gypsy, springer and beagle elements of *Drosophila;* and
- integrases of retroviruses RSV, SNV, Mo-MLV, MMTV, HTLV1 and HIV1;
and their fragments or variants thereof.

More preferably, the transposase is selected from the transposases able to form covalently joined SDRTS sequences such as sequences derived from procaryotic mobile elements IS1, IS2, IS3, IS5, IS10L, IS10L/R-2, IS26, IS30, IS50, IS150, IS186, IS911. In more preferred embodiment, said transposase is IS30 transposase from *Escherichia coli.*

Alternatively, the recombinase is a transposase selected from the transposases encoded by sequences able to form a covalently joined SDRTS sequences such as eukaryotic mobile elements Tc1 of *Caenorhabditis elegans,* the P and Copia elements of *Drosophila,* Ac-Ds Spm and Mu elements of maize, Ty1, Ty2, Ty3 elements of yeast, Tam1, Tam2 and Tam3 elements of *Antirrhinum,* Tx1 and Tx2 elements of *Xenopus.*

In another embodiment, the recombinase is a DNA recombinase selected from the group of site-directed recombinases comprising the Cre recombinase of bacteriophage P1, the FLP recombinase of *Saccharomyces cerevisiae,* the R recombinase of *Zygosaccharomyces rouxii* pSR1, the A recombinase of *Kluyveromyces drosophilarium* pKD1, the A recombinase of *Kluyveromyces waltii* pKW1, the integrase λ Int, the recombinase of the GIN recombination system of the Mu bacteriophage, the recombinase of the CIN recombination system of P1 bacteriophage, the recombinase of the MIN recombination system of P1-15 bacteriophage, the bacterial β recombinase, the invertase of the Hin system of *Salmonella,* the invertase of the FIM system of *Escherichia,* the integrase of the SLP1 system of *Streptomyces* or variants thereof.

The heterologous protein, or a fragment or a variant thereof comprises at least a DNA binding domain, said heterologous protein being selected among site-specific recombinases, transposases, procaryotic host factors, procaryotic activator proteins, procaryotic repressor proteins, eukaryotic transcription factors, DNA methylases, restriction endonucleases, chromatin proteins, or a fragment or a variant thereof. In a preferred embodiment, the heterologous protein is the procaryotic repressor protein cI of the lambda phage, or fragments or variants thereof. In another preferred embodiment, said heterologous protein is the eukaryotic zinc-finger transcription factor Gli, or fragments or variants thereof. In another preferred embodiment said heterologous protein is the Tet repressor, encoded by the tetracycline resistance gene, or fragments or variants thereof. In another preferred embodiment, said heterologous protein, or a fragment or a variant thereof is factor that binds DNA indirectly and is selected among proteins that are known to associate with DNA binding factor such as co-activators or co-repressors of transcription factors :
- such as TATA binding protein (TBP) associated factors (TAF_{IIS}) (Bell *et al*., 1999) ;
- factors associated with the RNA polymerase II Mediator complex, such as TRAPs (also called DRIPs or MEDs) (Malik *et al*., 2000) ;
- histone deacetylases (HDAC1, HDAC2, HDAC3 and HDAC8) (Exp. Cell. Res., 2001) ;
- histone acetyltransferases (GCN5, PCAF, p300, CBP, TIF2, SRC-1) (Marmorstein *et al*., 2001) ;
- histone methyltransferases (Jenuwein, 2001) ;
- transcription factors such as smad2 that do not bind DNA directly but in conjunction with other DNA binding factors of TGF beta signal transduction pathway.

It is also in the scope of the invention to use variants of the site-specific recombinase and the heterologous protein. The expression "variant" is understood to mean all the wild-type recombinases or heterologous protein, or fragments thereof, which may exist naturally and which correspond in particular to truncations, substitutions, deletions and/or additions of amino acid residues. These recombinases, heterologous proteins and fragments thereof are preferably derived from the genetic polymorphism in the population. The expression "variant" is also understood to mean the synthetic variants for which the above modifications are not naturally present, but were introduced artificially, by genetic engineering, or were chemically induced for example.

The DNA responsive element is preferably selected among operator regions of procaryotic repressors, methylation sites of sequence-specific methylases, recognition sites of restriction endonucleases, binding sites of host factors, recognition sequences of site-specific recombinases, hot spot sequences for transposases, transcription factor responsive elements (e.g. GC box of SP1 transcription factor, CAAT Box, octamer responsive element,...), the phase λ operator region, the CpG island, LHS, GOHS, IS30 recognition sequence, *(IS30)*_{*2*}. The heterologous protein comprising a DNA-binding domain that binds to the DNA responsive element is chosen accordingly. In a preferred embodiment, said DNA responsive element is the operator region of phage λcI repressor, and the heterologous protein is the procaryotic repressor protein cI of the lambda phage, and the variants thereof.

In one embodiment, the fusion protein of the invention comprises the cI repressor of the lambda phage or a fragment or a variant thereof (as a heterologous protein) and the IS30 transposase from *Escherichia coli* or a fragment or a variant thereof (as a transposase). In a more preferred embodiment, the fusion protein of the invention is of sequence SEQ ID N° 2.

In a second embodiment, the fusion protein of the invention comprises the DNA-binding domain of Gli transcription factor or a fragment or a variant thereof (as a heterologous protein) and the IS30 transposase from *Escherichia coli* or a fragment or a variant thereof (as a transposase). In a more preferred embodiment, the fusion protein of the invention is of sequence SEQ ID N°4.

In a third embodiment, the fusion protein of the invention comprises the Tet repressor or a fragment or a variant thereof (as a heterologous protein) and the IS30 transposase from *Escherichia coli* or a fragment or a variant thereof (as a transposase). In a more preferred embodiment, the fusion protein of the invention is of sequence SEQ ID N° 6.

In a preferred embodiment, the fusion protein of the invention comprises the IS30 transposase from *Escherichia coli* or a fragment or a variant thereof. In a more preferred embodiment, the fusion protein of the invention comprises the first 39 amino acids of the IS30 transposase.

By "fusion protein catalyzes recombination in the vicinity or at said DNA responsive element", it is meant that the donor DNA molecule is integrated in the DNA responsive element or at a distance smaller than 1500 bases pairs (bp), 1000 bp, 800 bp, 750 bp, 500 bp, 400 bp, 300 bp, 250 bp, 200 bp, 150 bp, 100 bp, 75 bp, 50 bp, 40 bp, 35 bp, 30 bp, 25 bp, 20 bp, 15 bp, 10 bp, or smaller than 5 bp.

The fusion protein of the invention can be produced by chemical synthesis or by a biological process. More preferably, the fusion protein of the invention is biologically produced by the means of recombinant DNA technologies in an organism that may be a higher organism (e.g. an animal), or a lower organism (e.g. a bacteria such as *Escherichia coli*, a yeast, a protozoan), a plant or cell derived from a multicellular organism such as fungus, an insect cell, a plant cell, a mammalian cell or a cell line, which carries an expression system as defined below.

The polypeptide produced as described above may be subjected to post-translational or post-synthetic modifications as a result of thermal treatment, chemical treatment (formaldehyde, glutaraldehyde etc.) or enzyme treatment (peptidases, proteinases and protein modification enzymes). As an example, glycosylation is often achieved when the polypeptide is expressed by a cell of a higher organism such as yeast or preferably a mammal. Glycosylation is normally found in connection with amino acid residues Asn, Ser, Thr or hydroxylysine. Such modifications of the protein fusion of the invention may be useful for example to enhance the half-life of this polypeptide in an organism, or in cells, to enhance the solubility of this polypeptide, or to facilitate the purification of the protein fusion of the invention (e.g. tagging).

As used herein, by "protein", "peptide" or "polypeptide" is meant any chain of amino acids, regardless of length or post-translational modification (e.g., glycosylation or phosphorylation).

The present invention also relates to the recombinant polynucleotide encoding for a protein fusion of the invention. In one embodiment, the invention provides recombinant polynucleotides of sequence SEQ ID N° 1, SEQ ID N° 3 and SEQ ID N° 5 encoding respectively for the fusion proteins of sequence SEQ ID N° 2, SEQ ID N° 4 and SEQ ID N° 6 of the invention.

Additionally, the invention provides a recombinant polypeptide comprising a polynucleotide selected among :
a) the polynucleotides of the invention ;
b) the polynucleotides presenting at least 70% identity after optimal alignment with the polynucleotide of step a) ;
c) fragments of polynucleotides of the invention encoding for a peptide having at least a site-directed recombinase activity;
d) the complementary sequence or RNA sequence corresponding to a polynucleotide of step a), b) or c).

As used herein, "percentage of identity" between two nucleic acids sequences or two amino acids sequences, means the percentage of identical nucleotides, respectively aminoacids, between the two sequences to be compared, obtained with the best alignment of said sequences, this percentage being purely statistical and the differences between these two sequences being randomly spread over the nucleic acids or aminoacids sequences. As used herein, "best alignment" or "optimal alignment", means the alignment for which the determined percentage of identity (see below) is the highest. Sequences comparison between two nucleic acids or amino acids sequences are usually realised by comparing these sequences that have been previously aligned according to the best alignment; this comparison is realised on segments of comparison in order to identify and compared the local regions of similarity. The best sequences alignment to perform comparison can be realised, beside by a manual way, by using the local homology algorithm developed by Smith and Waterman (1981), by using the local homology algorithm developed by Neddleman and Wunsch (1970), by using the method of similarities developed by Pearson and Lipman (1988), by using computer softwares using such algorithms (GAP, BESTFIT, BLAST P, BLAST N, FASTA, TFASTA in the Wisconsin Genetics software Package, Genetics Computer Group, 575 Science Dr., Madison, WI USA). To get the best alignment, one can preferably used BLAST software, with the BLOSUM 62 matrix, or the PAM or PAM 250 matrix. The identity percentage between two sequences of nucleic acids or amino acids is determined by comparing these two sequences optimally aligned, the nucleic acids or the aminoacids sequences being able to comprise additions or deletions in respect to the reference sequence in order to get the optimal alignment between these two sequences. The percentage of identity is calculated by determining the number of identical position between these two sequences, and dividing this number by the total number of compared positions, and by multiplying the result obtained by 100 to get the percentage of identity between these two sequences.

As used herein nucleic acids sequences having a percentage of identity of at least 70%, preferably, at least 72%, 75%, 77%, 80%, 82%, 85%, 87%, 90%, 92%, 95%, 97%, 98%, 99%, 99.5% after optimal alignment, means nucleic acids sequences having with regard to the reference sequence, modifications such as deletions, truncations, insertions, chimeric fusions, and/or substitutions, specially point mutations, the nucleic sequence of which presenting at least 70%, preferably, at least 72%, 75%, 77%, 80%, 82%, 85%, 87%, 90%, 92%, 95%, 97%, 98%, 99%, 99.5% identity after optimal alignment with the nucleic acid sequence of reference.

The invention also furnishes a DNA cassette comprising a promoter operably linked to a polynucleotide of the invention. A DNA cassette is a nucleic acid construct generated recombinantly or synthetically, with a series of specified nucleic acid elements which permit transcription of a particular nucleic acid in a target cell. The DNA cassette can be part of a vector or a nucleic acid fragment. Among these specified nucleic acid elements one can recite the "promoter" sequence which is a DNA regulatory region capable of binding RNA polymerase in a cell and initiating transcription of a downstream (3'direction) coding sequence. Within the promoter sequence will be found a transcription initiation site, as well as protein binding domains responsible for the binding of RNA polymerase. Eukaryotic promoters will often, but not always, contain TATA boxes and CAT boxes. Various promoters, including ubiquitous or tissue-specific promoters, and inducible and constitutive promoters may be used to drive the expression of the protein fusion gene of the invention. More preferably, the promoter is inducible by an inducing stimulus to cause the transcription of said fusion protein encoding gene or said polynucleotide.

"operatively linked" as used herein, includes reference to a functional linkage between a promoter and a second sequence (i.e. a polynucleotide of the invention), wherein the promoter sequence initiates and mediates the transcription of said DNA sequence corresponding to the second sequence. Generally, operatively linked means that the nucleic acid sequences being linked are contiguous and, where necessary to joint two protein coding regions, contiguous and in the same reading frame. According to the present invention, the nucleic acid sequences encoding for the site-specific recombinase protein (or a fragment or a variant thereof) and for the heterologous protein (or a fragment or a variant thereof) are operatively linked to generate a polynucleotide of the invention.

It is also a goal of the invention to furnish a vector comprising a polynucleotide of the invention (i.e. a cloning vector) or an expression vector comprising at least one DNA cassette of the invention. A " vector " is a replicon in which another polynucleotide segment is attached, so as to bring the replication and/or expression to the attached segment. Examples of vectors include plasmids, phages, cosmids, phagemid, yeast artificial chromosomes (YAC), bacterial artificial chromosomes (BAC), Phage P1 artificial chromosomes (PAC), human artificial chromosomes (HAC), viral vectors, such as adenoviral vectors, retroviral vectors, adeno-associated viral vectors and other DNA sequences which are able to replicate or to be replicated *in vitro* or in a host cell, or to convey a desired DNA segment to a desired location within a host cell. A vector according to the invention is preferably derived from a viral vector, more preferably an adenoviral, retroviral, or adeno-associated viral vector. The size of the vector is not critical. However it is preferably between about 5 and about 50 kilobases and more preferably between about 8 and about 20 kilobases. A vector can have one or more restriction endonuclease recognition sites at which the DNA sequences can be cut in a determinable fashion without loss of an essential biological function of the vector, and into which a DNA fragment can be spliced in order to bring about its replication and cloning. Vectors can further provide primer sites (e.g. for PCR), transcriptional and/or translational initiation and/or regulation sites, recombinational signals, replicons, selectable markers, etc. The vector can further contain a selectable marker suitable for use in the identification of cells transformed with the vector. The vector is either a circular or linear molecule.

The fusion gene encoding the fusion protein of the invention present in a DNA cassette or in an expression vector can be expressed using any suitable expression sequences. Numerous expression sequences are known and can be used for expression of the fusion gene. Expression sequences can generally be classified as promoters, terminators, and, for use in eukaryotic cells, enhancers. Expression in prokaryotic cells also requires a « *Shine-Dalgarno »* sequence just upstream of the coding region for proper translation initiation. Promoters suitable for use with prokaryotic hosts illustratively include the β-lactamase and lactose promoter systems, tetracycline (tet) promoter, alkaline phosphatase promoter, the tryptophan (trp) promoter system and hybrid promoters such as the tac promoter. However, other functional bacterial promoters are suitable. Their nucleotide sequences are generally known. Suitable promoting sequences for use with yeast hosts include, for example, the promoters for 3-phosphoglycerate kinase, enolase, glyceraldehyde-3-phosphate dehydrogenase, hexokinase. Examples of inducible yeast promoters suitable for use in the vectors of the invention include, for example, the promoter regions for alcohol dehydrogenase 2, isocytochrome C, acid phosphatase. Yeast enhancers also are advantageously used with yeast promoters. Preferred promoters for use in mammalian host cells include promoters from polymoma virus, Simian Virus 40 (SV40), adenovirus, retroviruses, *hepatitis* B virus, *herpes simplex* virus (HSV), Rous *sarcoma* virus (RSV), mouse mammary tumor virus (MMTV), and most preferably cytomegalovirus (CMV), or from heterologous mammalian promoters such as the β-actin promoter. Transcription of the fusion protein gene or the polynucleotide of the invention by higher eukaryotes can be increased by inserting an enhancer sequence into the vector. Many enhancer sequences are now known from mammalian genes (globin, elastase, albumin, and insulin) or from eukaryotic cell virus (SV40, CMV). The disclosed vectors preferably also contain sequences necessary for accurate 3'end termination ; in eukaryotic cells, this would be a polyadenylation signal. In prokaryotic cells, this would be a transcription terminator.

The recombinant DNA technologies used for the construction of the vectors according to the invention are those known and commonly used by persons skilled in the art. Standard techniques are used for cloning, isolation of DNA, amplification and purification; the enzymatic reactions involving DNA ligase, DNA polymerase, restriction endonucleases are carried out according to the manufacturer's recommendations. These techniques and others are generally carried out according to Sambrook *et al.* (1989).

The polynucleotide (in DNA or RNA form), the vector or fragments thereof may be introduced into the host cell by standard methods as described below. According to another embodiment of the invention, the fusion protein can be directly introduced into the organism, or into a cell of the organism. This introduction can be carried out by injection into a tissue or an organ in the case of an organism, or by microinjection in the case of a cell.

The disclosed vectors can be used to transiently transfect or transform host cells, or can be integrated into the host cell chromosome. Preferably, however, the vectors can include sequences that allow replication of the vector and stable or semi-stable maintenance of the vector in the host cell. Many such sequences for use in various cells (that is, eukaryotic and prokaryotic cells) are known and their use in vectors routine. Generally, it is preferred that replication sequences known to function in host cells of interest be used.

More preferably, the invention provides a gene-targeting vector comprising at least:
a) A gene encoding a fusion protein or a polynucleotide of the invention;
b) Optionally, a promoter that is operatively linked to said fusion protein gene or to said polynucleotide;
c) Optionally, a DNA responsive element recognized by the DNA binding domain of said fusion protein ;
d) At least two SDRTS recognized by the recombinase domain of said fusion protein ;
e) At least one transposable DNA sequence of interest, wherein said DNA sequence of interest is located between said two SDRTS sequences ;
f) Optionally, at least one marker gene; and wherein one of said SDRTS is located between said fusion protein encoding gene or said polynucleotide and said DNA sequence of interest.

In another embodiment, the invention provides gene targeting vector comprising at least:
a) A gene encoding a fusion protein or a polynucleotide of the invention ;
b) Optionally, a promoter that is operatively linked to said fusion protein gene or to said polynucleotide;
c) At least one DNA responsive element recognized by the DNA binding domain of said fusion protein ;
d) At least two SDRTS recognized by the recombinase domain of said fusion protein, said SDRTS being joined covalently together and being separated at most by 1000 bp, 750 bp, 500 bp, 250 bp, 200 bp, 150 bp, 100 bp, 75 bp, 50 bp, 40 bp, 30 bp, 25 bp, 20 bp, 18 bp, 15 bp, 14 bp, 13 bp, 12 bp, 11 bp, 10 bp, 9 bp, 8 bp, 7 bp, 6 bp, 5 bp, 4 bp, 3 bp, 2 bp, 1 bp, and more preferably at most by 20 bp.
e) Optionally, at least one marker gene.

In another embodiment, the invention provides a gene targeting vector comprising:
a) Optionally, a DNA responsive element recognized by the DNA binding domain of a fusion protein of the invention ;
b) At least two SDRTS recognized by the recombinase domain of said fusion protein of step a);
c) At least one transposable DNA sequence of interest, wherein said gene is located between said two SDRTS sequences ;
d) Optionally, at least one marker gene.

As used herein, "marker gene" means a gene that encodes a protein or a peptide (i.e. a selectable marker) that allows one to select for or against a molecule or a cell that contains it, often under particular conditions i.e. in presence of a selective agent. These marker proteins include but are not limited to products which provide resistance against otherwise toxic compounds (e.g., antibiotics). For example, the ampicillin or the neomycin resistance genes constitute genes encoding for selection marker of the invention. Those selection markers can be either positive or negative (see Capecchi *et al*., US 5 631 153). According to a preferred embodiment, said selection marker protein is a positive selection marker protein encoded by a gene selected in the group consisting of antibiotic resistance genes, hisD gene, *Hypoxanthine phosphoribosyl transferase* (HPRT) gene, guanine-phosphoribosyl-transferase (Gpt) gene. Said antibiotic resistance gene is selected in the group consisting of hygromycin resistance genes, neomycin resistance genes, tetracyclin resistance genes, ampicillin resistance genes, kanamycin resistance genes, phleomycin resistance genes, bleomycin resistance genes, geneticin resistance genes, carbenicillin resistance genes, chloramphenicol resistance genes, puromycin resistance genes, blasticidin-S-deaminase genes. In a preferred embodiment, said antibiotic resistance gene is a hygromycin resistance gene, preferably an *Escherichia coli* hygromycin-B phosphotransferase (hpt) gene. In this case, the selective agent is hygromycin. In another preferred embodiment, said antibiotic resistance gene is a neomycin resistance gene. Said neomycin resistance gene is chosen in respect to the cellular host in which the fusion protein is expressed. For an expression restricted to prokaryotic cells, the neomycin resistance gene encoded by the Tn10 transposon is preferred; in that case, the selective agent is kanamycin. More preferably, the Tn5 neomycin resistance gene is used; such gene allowing to perform the selection both in eukaryotic and procaryotic cells. In this latter case, the selective agent is G418. In another embodiment, the positive selection marker protein of the invention is His D; in that case, the selective agent is Histidinol. In another embodiment, the positive selection marker protein of the invention is Hypoxanthine phosphoribosyl transferase (HPRT) or Hypoxanthine guanosyl phosphoribosyl transferase (HGPRT); in that case, the selective agent is Hypoxanthine. In another embodiment, the positive selection marker protein of the invention is guanine-phosphoribosyl-transferase (Gpt); in that case, the selective agent is xanthine. It is also in the scope of the invention to use negative selection marker proteins. For example, the genes encoding for such proteins are the HSV-TK gene ; in that case the selective agent is Acyclovir-Gancyclovir. For example, the genes encoding for such proteins are the Hypoxanthine phosphoribosyl transferase (HPRT) gene or the guanine-phosphoribosyl-transferase (Gpt) gene ; in these cases, the selective agent is 6-Thioguanine. For example, the gene encoding for such proteins is the cytosine deaminase; in that case the selective agent is 5-fluoro-cytosine. Other examples of negative selection marker proteins are the viral and bacterial toxins such as the diphteric toxin A (DTA). The marker gene can also be a "reporter gene". By "reporter gene" is meant any gene which encodes a product whose expression is detectable. A reporter gene product may have one of the following attributes, without restriction : fluorescence (*e.g*., green fluorescent protein), enzymatic activity (*e.g*., lacZ or luciferase), or an ability to be specifically bound by a second molecule (*e.g*., biotin or an antibody-recognizable epitope).

The transposable DNA sequence of interest is any DNA molecule. It can either be a genomic DNA fragment, such as gene(s), intron(s), exon(s), regulatory sequence(s)or combinations or fragments thereof, or a recombinant DNA molecule such as a cDNA gene for instance. According to a preferred embodiment, the transposable DNA sequence of interest is a therapeutic gene. A "therapeutic gene" is a gene that corrects or compensates for an underlying protein deficit or, alternately, that is capable of down-regulating a particular gene, or counteracting the negative effects of its encoded product, in a given disease state or syndrome. Moreover, a therapeutic gene can be a gene that mediates cell killing, for instance, in the gene therapy of cancer. According to another embodiment, the transposable DNA sequence of interest is a reporter gene as previously defined.

The present invention also provides a kit to perform site-directed recombination ; such kit comprises at least :
(i) a gene encoding a fusion protein, a polynucleotide, optionally a promoter that is operatively linked to said fusion protein gene or to said polynucleotide, a DNA cassette or a vector of the invention ; and
(ii) a donor DNA molecule that comprises a transposable DNA sequence of interest, said DNA sequence of interest being flanked at its 5' and/or 3' ends by said SDRTS, said SDRTS being specifically recognized by the recombinase domain of the fusion protein encoded by a gene, a polynucleotide, a DNA cassette, a vector of step (i) ;
(iii) optionally, a recipient DNA molecule that comprises at least one DNA responsive element that binds the DNA-binding domain of the fusion protein encoded by a gene, a polynucleotide, a DNA cassette or a vector of (i).

The invention also provides a kit to perform site-directed recombination wherein said kit comprises at least :
(i) a fusion protein according to the invention ; and
(ii) a donor DNA molecule that comprises a transposable DNA sequence of interest, said DNA sequence of interest being flanked at its 5' and/or 3' ends by said SDRTS, said SDRTS being specifically recognized by the recombinase domain of the fusion protein of (i) ;
(iii) optionally, a recipient DNA molecule that comprises at least one DNA responsive element that binds the DNA-binding domain of the fusion protein of (i) .

In another embodiment, the invention provides a kit to perform site-directed recombination wherein said kit comprises at least :
(i) a gene targeting vector of the invention ; and
(ii) optionally, a recipient DNA molecule that comprises at least one DNA responsive element that binds the DNA-binding domain of the fusion protein of (i).

It is also a goal of the invention to provide a method for *in vitro* site-directed DNA recombination, said method comprising the steps of combining :
a) a donor DNA molecule that comprises a transposable DNA sequence of interest, the DNA sequence of interest being flanked at its 5' and/or 3' ends by said SDRTS being specifically recognized by the recombinase domain of the fusion protein of the invention ; with
b) a recipient DNA molecule that comprises at least one DNA responsive element that binds the DNA-binding domain of the fusion protein of the invention ; with
c) a fusion protein of the invention.

In another embodiment, the invention provides a method for *in vivo* site-directed DNA recombination, said method comprising the steps of combining into a cell:
a) a donor DNA molecule that comprises a transposable DNA sequence of interest, the DNA sequence of interest being flanked at its 5' and/or 3' ends by SDRTS being specifically recognized by the recombinase domain of the fusion protein of the invention ; with
b) a recipient DNA molecule that comprises at least one DNA responsive element that binds the DNA-binding domain of the fusion protein of the invention ; with
c) a fusion protein, or a gene encoding a fusion protein or a polynucleotide, optionally a promoter that is operatively linked to said fusion protein gene or to said polynucleotide, or a DNA cassette, or a vector of the invention, said gene or polynucleotide being efficiently expressed in said cell.

More preferably, the DNA recombination is selected among DNA insertion, deletion, inversion, translocation and fusion.

By "transposon" or "transposable element" is meant a linear strand of DNA capable of integrating into a second strand of DNA which may be linear or may be a circularized plasmid. The transposable elements of the invention have SDRTS sequences at their extremities, and are able to site-specifically integrate into sites within the second strand. SDRTS sequences of the present invention have preferably less than 100 bp, 80 bp, 75 bp, 50 bp, 45 bp, 40 bp, 35 bp, 30 bp, 25 bp, 20 bp, 15 bp, 12 bp, 10 bp, 8 bp, less than 5 bp. Preferred transposable elements of the invention have a short (*e.g*., less than twenty) base pair repeat at either end of the linear DNA. However, SDRTS sequences longer than 50 bp are also in the scope of the invention. The SDRTS sequences of the invention are generally inverted repeats of one another (exact or closely related). According to the present invention, preferred SDRTS include the 26 bp long inverted repeat ends of IS30 or the appropriate SDRTS sequences of transposases and recombinases as above listed.

By "recipient DNA molecule" is meant any DNA molecule. Preferably, this recipient DNA molecule is a cellular genome, such as bacterial chromosome(s), or eukaryotic chromosome(s). Alternatively, this recipient DNA molecule is a viral genome. The recipient DNA molecule can be a naked DNA molecule or have a chromatin structure. The recipient DNA molecule is either present in a living cell or in a cell free extract.

The invention also provides a method for the stable introduction of a DNA sequence of interest into at least one recipient DNA molecule of a cell, said recipient DNA molecule comprises at least one DNA responsive element that binds the DNA-binding domain of the fusion protein of the invention, and said method comprising the steps of :
a) providing a donor DNA molecule that comprises a transposable DNA sequence of interest, the DNA sequence of interest being flanked at its 5' and/or 3' ends by said SDRTS being specifically recognized by the recombinase domain of the fusion protein of the invention ;
b) introducing into the cell said donor DNA molecule; and,
c) previously, simultaneously, or separately, introducing into said cell a fusion protein or a gene encoding a fusion protein or a polynucleotide, optionally a promoter that is operatively linked to said fusion protein gene or to said polynucleotide, or a DNA cassette, or a vector, said gene or polynucleotide being efficiently expressed in said cell.

The targeting vector of the invention functions as a functional transposon or a transposable element by allowing the transformation and/or the integration of at least a DNA sequence of interest into a prokaryotic or eukaryotic host cell genome. In a preferred embodiment, the vector or transposable element of the invention comprises a gene encoding the fusion protein, two SDRTS sequence, a DNA sequence of interest, where the DNA sequence of interest is located between the two SDRTS sequences, and a promoter that is adapted to cause the transcription of the fusion protein in a temporal, spatio-controlled, inducible or constitutive manner; where the fusion protein excises from the vector a fragment comprising the two SDRTS sequences and the portion of the vector between the two SDRTS sequences, and to insert the excised fragment into a chromosome or a DNA sequence of the host cell. This arrangement insures that the transposase gene is not incorporated into the target chromosome or the DNA sequence of the host cell, insuring that the transformation will be stable. Descendants of a cell transformed with the vector will not have a copy of the transposase gene. Without a transposase gene to encode a transposase, there will be nothing to promote excision of the DNA sequence of interest from the genome.

It is also a goal of the invention to provide a host cell transformed by at least one polynucleotide, one DNA cassette, one vector of the invention.

A "host," as the term is used herein, includes prokaryotic or eukaryotic organisms that can be genetically engineered. For examples of such host cells, see Sambrook *et al*., 1989. A host cell of the invention is either a prokaryotic cell or an eukaryotic cell derived from said organisms. The cell of the invention is characterized by the fact that the protein fusion encoded by said polynucleotide, said DNA cassette, or said vector, is expressed and/or is biologically active in said cell. By "biologically active", it is meant that the fusion protein of the invention, or a fragment or a variant thereof, is able to catalyse site-directed DNA recombination.

In a preferred embodiment, the cell of the invention is selected among eukaryotic and prokaryotic cells. Preferably, the cell of the invention is an eukaryotic cell selected among selected among mammalian cells (e.g. human cells, murine cells), yeast cells, amphibian cells, fish cells, *Drosophila* cells, *Caenorhabditis* cells, plant cells. More preferably, it is a mammalian cell, more specifically selected among totipotent stem cells (e.g. embryonic stem cells), multipotent stem cells, fibroblast, cardiac muscle cells, skeletal muscle cells, glial cells, neurons. In a preferred embodiment, it is a murine embryonic stem cells (ES cells). In another embodiment, the cell of the invention is a prokaryotic cell selected among *Escherichia sp., Bacillus sp., Campylobacter sp., Helicobacter sp., Agrobacterium sp., Staphylococcus sp., Thermophilus sp., Azorhizobium sp., Rhizobium sp., Neisseria sp., Neisseria sp., Pseudomonas sp., Mycobacterium sp., Strepkomyces sp., Corynebacterium sp., Lactobacillus sp., Micrococcus sp., Yersinia sp., Brucella sp., Bortadella sp., Proteus sp., Klebsiella sp., Erwinia sp., Vibrio sp., Photorhabdus sp., desulfovibrio sp., Listeria sp., Clostridium sp., Actynomyces sp., Haemophilus sp..*

Host cells can be transformed with the disclosed polynucleotides, DNA cassette, or vectors of the invention using any suitable means and cultured in conventional nutrient media modified as is appropriate for inducing promoters, selecting transformants of detecting expression. Suitable culture conditions for host cells, such as temperature and pH, are well known. In a preferred embodiment, transformed cells of the invention are cultured in presence of selective agent as previously described in the cell culture media.

Such polynucleotides, DNA cassette, or vectors of the invention can be introduced into a host cell either *in vivo* or *in vitro* using known techniques depending of the nature of the host cells. DNA can be introduced into recipient bacteria and yeast in a number of ways. These include conjugation, transformation, transduction, or the most recent development, electroporation. Transformation is the introduction of naked DNA into competent recipient cells. Competence may be either naturally or artificially, induced using calcium or rubidium chloride. Electroporation uses pulses of high voltage electric current to enable uptake of DNA into cell protoplasts. In transduction, bacteriophages, encapsidate foreign DNA in place of their genome which is introduced into the recipient in a subsequent round of phage infection. DNA can be introduced into eukaryotic cells, preferably into mammalian cells by using known techniques such as CaPO₄ precipitation, electroporation, cationic lipofection, use of artificial viral envelopes, direct injection (e.g., intravenous, intraperitoneal or intramuscular), and micro-injection into a zygote or a pronucleus of a zygote. Thus, the invention relates to an isolated transgenic host cell transformed by a polynucleotides, DNA cassette, or vectors of the invention; the protein fusion encoded by said polynucleotide, DNA cassette, or vector of the invention, is expressed and biologically active in said cell of the invention.

The present invention also relates to the transgenic organism, comprising at least one cell according to the invention. An "organism", as the term is used herein, includes but is not limited to, bacteria, yeast, animal, plants. Among the animals, one can designated mammals, such as rodent, primates, excepted humans, farm animals. In a preferred embodiment, the animal is a mouse, a rat, a Chinese pig, a hamster, a rabbit, a pig, a cow, a horse, a goat, a sheep. In a preferred embodiment, the animal is a mouse. In another preferred embodiment, the organism is a yeast.

Therefore the invention relates to the use of a fusion protein, a polynucleotide, a vector, a DNA cassette, or a kit of the invention, for producing transgenic cells and/or transgenic animals. Transgenic cells and animals are among the most useful research tools in the biological sciences. These cells and animals have an heterologous (*i.e*., foreign) gene, or gene fragment, incorporated into their genome that is passed on to their offspring. Although there are several methods of producing transgenic animals, the transgenic cells and organisms of the invention are generally obtained by using microinjection of a polynucleotide, a vector, a DNA cassette of the invention into single cell embryos. These embryos are then transferred into pseudopregnant recipient foster mothers. The offspring are then screened for the presence of the new gene, or gene fragment. Potential applications for transgenic animals include discovering the genetic basis of human and animal diseases, generating disease resistance in humans and animals, gene therapy, drug testing, and production of improved agricultural livestock. More specifically, the use of a fusion protein, a polynucleotide, a vector, a DNA cassette, a kit of the invention is useful to perform gene targeting, gene knock-out (KO), gene knock-in (KI), gene trapping (e.g. PolyA-trapping, exon trapping, promoter trapping, enhancer trapping), or transposon tagging.

By "gene targeting" is meant a process whereby a specific gene, or a fragment of that gene, is altered. This alteration of the targeted gene may result in a change in the level of RNA or protein that is encoded by that gene, or the alteration may result in the targeted gene encoding a different RNA or protein than the untargeted gene. The targeted gene may be studied in the context of a cell, or, more preferably, in the context of a transgenic animal.

By "Knock-out" is meant an alteration in the nucleic acid sequence that reduces the biological activity of the polypeptide normally encoded therefrom by at least 80% compared to the unaltered gene. The alteration may be an insertion, deletion, frameshift mutation, or missense mutation. Preferably, the alteration is an insertion or deletion, or is a frameshift mutation that creates a stop codon.

By "Knock-in" is meant an alteration in the nucleic acid sequence by insertion of an additional exogenous DNA molecule that allows either an increased biological activity of the polypeptide normally encoded therefrom compared to the unaltered gene or the expression of an heterologous protein encoded by said heterologous DNA molecule usually under the control of the regulatory elements of the endogenous gene encoding the polypeptide. The "Knock-in" strategy may be used to insert a reporter gene (*e.g*., green fluorescent protein), the expression of which is then subjected to the identical regulatory controls that were placed on the gene that was replaced. This is useful for tagging particular cell types or to facilitate studies of gene regulation. Alternatively, gene replacement may be used to assess the degree of functional redundancy among two related proteins or to examine the phenotype produced by replacing a normal protein with a mutated form.

"Gene-trapping" is an approach based on a class of reporter vectors, that have been designed containing a splice-acceptor site upstream of a reporter gene (lacZ, gfp, neoR). Integration of these vectors into a genomic locus downstream of a functional promoter results in the generation of a fusion transcript between the endogenous gene and the reporter gene. Fusion transcripts from insertion of these vectors mimic endogenous gene expression at the insertion locus. This expression can be monitored by visualizing reporter gene activity (Gossler *et al*., 1989). Usually the gene trap vectors will also act as insertional mutagens, disrupting the endogenous gene function.

According to the integration locus, one can distinguish different gene-trap approaches named "exon trapping", "promoter trapping", "enhancer trapping".

For instance, enhancer trapping involves the random insertion into an eukaryotic genome of a promoter-less foreign gene (the reporter) whose expression can be detected at the cellular level. Expression of the reporter gene indicates that it has been fused to an active transcription unit or that it has inserted into the genome in proximity to cis-acting elements that promote transcription. This approach is important in identifying genes that are expressed in a cell type-specific or development stage-specific manner.

The above system can be a tool for the genetic modification of both prokaryotic and eukaryotic cells and organisms. The system allows site-specific integration of genetic information in whole genomes resulting in altered genotypes (gain of function mutation or loss of function mutation). A gain of function mutation (e.g. gene therapy, *knock-in* or transgenesis) is achieved if the targeting vector contains a coding sequence for a given protein, or sequences that induce the transcriptional activity of a gene adjacent to the integration site in the recipient genome. A loss of function genotype (e.g. insertional mutagenesis) is created when the targeted insertion results in the transcriptional or translational inactivation of the gene adjacent to the integration site. The technology will allow efficient targeting of DNA sequences in genomes where other site-specific recombination or gene targeting technologies are not feasible.

It is proposed that eukaryotic systems (including commonly used models such as yeast, plants, animal models such as *C. elegans, Drosophila,* teleost fish, *Xenopus,* as well as chick or mammalian systems) or any other cell systems in which the technology for the introduction of foreign DNA is available could utilize a site-targeted recombination system as described in this invention. Utilization of such a system is not restricted to *in vivo* applications, but is extendable to *in vitro* experimentation utilizing cell culture (e.g. embryonic stem cells).

Accordingly, the methods, DNA molecules and vectors of the invention can be used for a variety of therapeutic and diagnostic applications which require stable and efficient integration of transgene sequences into genomic DNA of cells. The methods, polynucleotides and vectors can be used to transform a wide variety of eukaryotic cells (e.g., mammalian) cells and provide the advantage of high efficiency DNA transfer.

Therefore, the present invention provides vector of the invention as medicament. Such vector as a medicament is useful to perform a gene therapy to a patient in need of such treatment (e.g. cancer, metabolic diseases, infectious diseases, genetic diseases...).

Unless defined otherwise, all technical and scientific terms used herein have the same meaning as is commonly understood by one of the skill in the art to which this invention belongs.

The figures and examples presented below are provided as further guide to the practitioner of ordinary skill in the art and are not to be construed as limiting the invention in anyway.

### EXAMPLES

The inventors used the teleost fish zebrafish (*Danio rerio*) in their experiments as a vertebrate animal model to address the suitability of the targeted transposition system to vertebrate genetics. While transposon mediated recombination systems have previously been shown to be active in fish (Raz *et al*., 1998 ; Lam *et al*., 1996 ; Kawakami *et al*., 2000 ; Izsvak *et al*., 1995 ; Ivics *et al*., 1997), these systems either proved to be inefficient or were burdened by the restricted size of donor DNA that can be inserted by these 'cut and paste' transposases. In the present invention, the inventors propose to use a transposition based recombination system which does not restrict the size of donor DNA to be inserted into the target genome. The IS30 mediated transpositional integration will be more efficient than the existing transposase based recombination systems.

### EXAMPLE 1 : CONSTRUCTION AND TEST OF A SIMPLE TRANSPOSITION REACTION IN ZEBRAFISH EMBRYOS

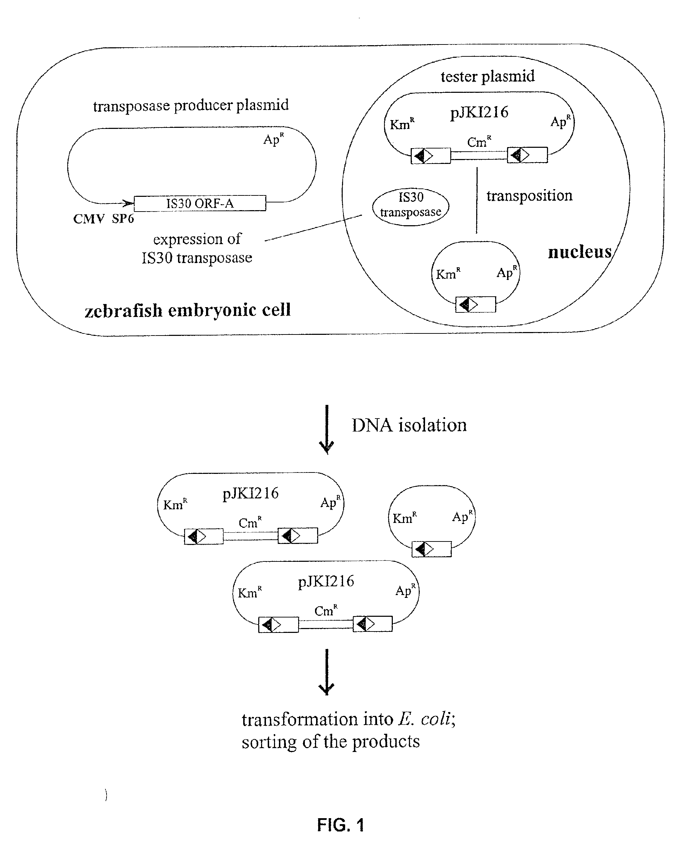

In this part of the project, evidence is provided that the prokaryotic IS30 element can induce transposition in a eukaryotic system. A test system based on an IS30-specific transpositional reaction has been established. In the system the transposase gene is separated from its active site (the *(IS30)*_{*2*} structure) and cloned in different plasmids in order to test their activity. The system contained the following components:
- **Tester plasmid:** the plasmid contains two intermediate dimer inverted repeat elements *(IS30)*_{*2*} separated by a chloramphenicol resistance gene (CmR) but without an active transposase gene (Farkas *et al*., 1996). The transposition reaction results in the specific loss of the CmR gene, which can be detected by replica plating (Fig. 1).
- **Transposase producer construct:** the open reading frame of the IS30 transposase was PCR amplified, cloned into the vector pBluescriptSK (Stratagene) and sequenced in order to verify the integrity of the transposase. In order to place the IS30 transposase ORF under the control of a well-characterised eukaryotic promoter (*CMV*) the transposase ORF was cloned as a XhoI cassette into various pCS2 derivative plasmid vectors (Turner *et al*., 1994). Several IS30 transposase producer constructs have been developed where the IS30 transposase is fused to signal peptides to allow the detection of the transposase protein produced in fish embryos. Further, some of these constructs carry a sequence encoding an eukaryotic nuclear localization signal peptide (NLS) in order to introduce the synthesized protein into the cell nucleus:

**Table I :**

| **Transposase producer constructs** | | |
|---|---|---|
| **Name** | **Vector used Description** | |
| IS30 | CS2 | IS30 ORF alone |
| IS30+MT | CS2 + MT | IS30 ORF N-terminal part was fused to the myc epitope tag (MT) in order to allow the immuno-histochemical detection of the fusion protein in fish cells |
| IS30+NLS | CS2 + NLS | IS30 ORF N-terminal part was fused to the nuclear localization signal (NLS) in order to transfer the synthesized protein to the nucleus |
| IS30+NLSMT | CS2 +NLSMT | IS30 ORF N-terminal part was fused both to the myc tag MT and the NLS |

The inventors have verified previously that the eukaryotic promoter CMV retained its activity in *Escherichia coli* (unpublished results), hence providing a possibility to test the transposition activity of the constructs described above in bacteria. *E. coli* cells carrying the tester plasmid together with one of the transposase producer plasmids were selected for the kanamycin resistance marker (Km^{R}) of the vector. The detection of transposition was based on the loss of the Cm^{R} marker gene (CmS) as described previously (Farkas *et al*., 1996). The results indicated that the fusion proteins were active in transposition (Table II).

**Table II :**

| **Transposition of the transposase producer constructs in *E. coli*** | | | | | | |
|---|---|---|---|---|---|---|
| **Constructions** | **Total tested** | **Rearranged products** | | **Transposition** | **Deletion** | **Other** |
| | | **CmS** | **%** | | | |
| tester | 1986 | 0 | <0.05 | | | |
| tester+IS30 | 1324 | 23 | 1.74 | 20 | 2 | |
| tester + NLS IS30 | 1546 | 18 | 1.16 | 16 | 1 | 1 |
| tester+MT IS30 | 1765 | 14 | 0.79 | 14 | | |
| tester+ NLS MTIS30 | 1878 | 21 | 1.12 | 19 | 1 | 1 |

The frequency of transposition was relatively low compared to the values obtained by an efficient prokaryotic promoter, but the majority of the recovered recombined plasmids were products of transposition (specific loss of the Cm^{R} gene). In the control experiment where only the tester was used, no transposition reaction was detected. There was no significant difference between the activities of the transposase producer constructs (IS30, NLSIS30, MTIS30, NLSMTIS30) suggesting that the N-terminal fusion of peptides does not effect the activity of the transposase protein.

### EXAMPLE 2 : DETECTION OF TRANSPOSITIONAL DELETION GENERATED BY IS30 TRANSPOSASE IN ZEBRAFISH

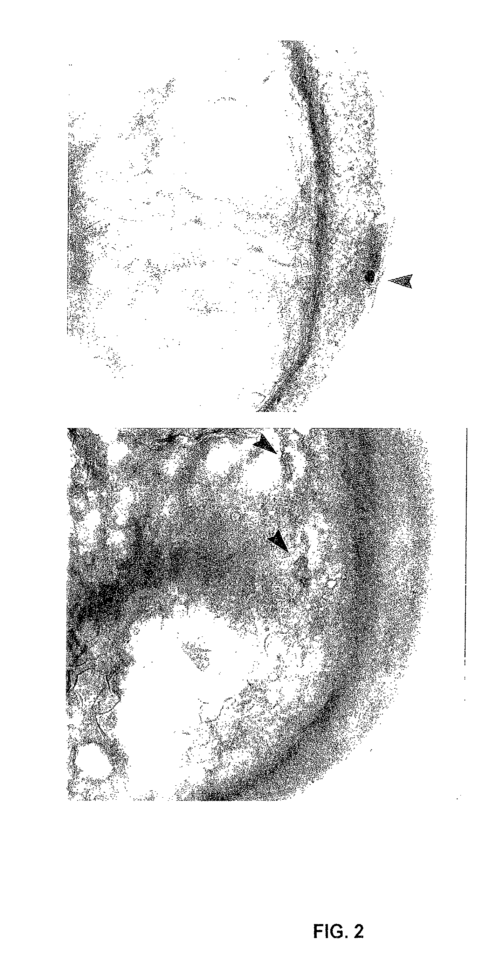

Zebrafish embryos were co-injected with the tester plasmids together with different transposase producers. Immunohistochemical staining was applied to detect the MYC epitope tagged IS30 transposase protein in fish embryos. Synthesis and localisation of the protein was demonstrated in nuclei as well as in the cytoplasm of cells in mRNA-injected zebrafish embryos (Fig. 2). It was concluded that the transposase protein was synthesized in the cells *in vivo*. After 10 hours of development (neurula stage) total DNA was purified from the embryos and this DNA was used to transform *E. coli* cells in order to detect transposition of plasmid sequences in fish embryos. The same method was used as described above and in Farkas *et al.* (1996). The results indicated that IS30 type transposition reaction occurred in fish embryos (Table III).

**Table III :**

| **Transpositional deletion by IS30 transposase in zebrafish** | | | | | | |
|---|---|---|---|---|---|---|
| **Constructions** | **total tested** | **rearranged products** | | **rearranged products** | | |
| | | **CmS** | **%** | **transposition** | **deletion** | **other** |
| control non injected | 0 | 0 | | | | |
| tester | 1949 | 2 | 0.1 | 1 | 1 | |
| tester + IS30 | 1680 | 15 | 0.89 | 12 | 2 | 1 |
| tester + NLS IS30 | 1718 | 14 | 0.81 | 10 | 1 | 3 |
| tester + NLSMTIS30 | 516 | 4 | 0.78 | 4 | | |

The reaction catalyzed by the transposase in embryos resulted in the specific loss of the marker gene, therefore the transformed bacteria exhibited the kanamycin resistance (Km^{R}), but not the chloramphenicol resistance (Cm^{R}) marker. When the tester alone was injected into the embryos (negative control), the frequency of the observed rearrangements was low (0.1 %). In contrast, the frequency of recombinations was significantly higher (app. 8 times difference) when one of the transposase producer constructs was co-injected. Moreover, the majority of the recovered plasmids contained the expected transpositional deletion product (Fig. 1), which was confirmed by sequencing. The remaining recovered isolates were products of deletions or multiple rearrangements. All of these results confirm that the IS30 element was active in zebrafish. There was no significant difference between the activities of the different transposase producer constructs used.

The transposase producer constructs contain a promoter to allow the *in vitro* synthesis of IS30 mRNA. *in vitro* transcribed IS30 mRNAs were injected into the fish embryos. To test activity of the transposase in zebrafish embryos, total DNA was isolated from injected embryos and loss of the Cm^{R} marker gene from the tester plasmid was assayed after transformation of *E. coli* as described previously. The results indicated that the injected IS30 transposase mRNA was translated and correctly excised the Cm^{R} marker gene in fish embryos at a relatively high frequency (Table IV).

**Table IV :**

| **Transpositional deletion by IS30 transposase injected as mRNA in zebrafish** | | | | | | |
|---|---|---|---|---|---|---|
| **Constructions** | **total tested** | **rearranged products** | | **rearranged products** | | |
| | | **CmS** | **%** | **transposition** | **deletion** | **other** |
| control non injected | 0 | 0 | | | | |
| tester | 1848 | 1 | 0.05 | | 1 | |
| tester + IS30mRNS | 907 | 3 | 0.33 | 2 | | 1 |
| tester + NLS IS30 mRNA | 787 | 13 | 1.65 | 11 | 1 | 1 |
| tester + MT IS30 mRNA | 38 | 2 | 5.2 | 2 | | |
| tester + NLSMTIS30 mRNA | 282 | 3 | 1.06 | 3 | | |

The embryos injected with the tester alone yielded very low frequencies of recombined products (0.05%), however, when the transposase was also co-injected as mRNA the frequency of recombination was significantly increased by up to 100-fold (0.33-5.2 %). Altogether the results indicated that *in vitro* synthesised mRNA can be used to produce active transposase in the fish embryo.

### EXAMPLE 3 : TRANSPOSITIONAL INSERTIONS BY IS30 TRANSPOSASE IN ZEBRAFISH

The above described assay is based on a specific reaction (characteristic for IS30) i.e. on the site-specific deletion of DNA fragments flanked by dimerized inverted repeats *(IS30)*_{*2*} by the IS3- transposase. In order to detect an integration reaction resulting in insertion of donor sequences into a recipient DNA, the prerequisite for transgene integration induced by IS30 in the zebrafish genome, a second set of experiments were performed. To detect transpositional integration reactions induced by IS30, the following experimental setup was developed.

### 3.1. Constructs of a gene trap- insertion system

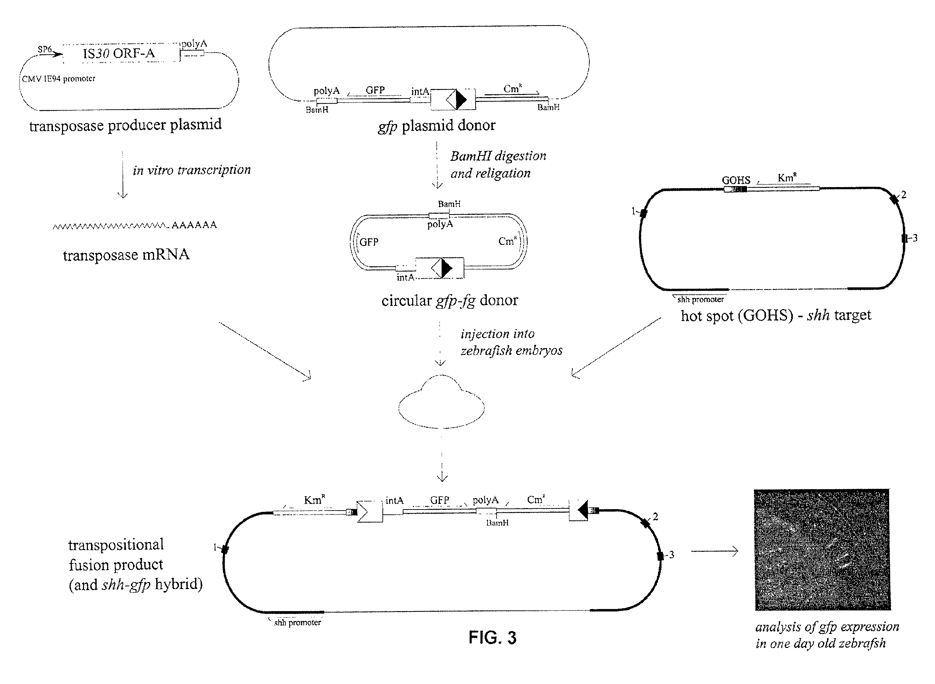

A gene trap construct has been generated which contains a *gfp* reporter gene attached to a splice-acceptor sequence to allow the expression of the marker gene only in the case of a gene-trap event, i.e. when the *gfp* gene is inserted into an intronic sequence of a gene (Skarnes, 1990). The integration of the gene trap construct into a transcribed region of the fish genomic DNA is expected to be facilitated by IS30 transposase which recognizes the *(IS30)*_{*2*} dimer intermediate structure of the donor DNA. The recombination of the gene trap donor and an intronic target results in the expression of the *gfp* reporter gene, allowing the rapid detection of insertion into a genomic gene. In order to detect the integration of a marker gene into a genomic gene in the zebrafish embryo a test system was developed where the intronic integration in a given gene is modeled by using a target plasmid which contains an intact genomic gene (see schematic in Fig. 3).

The test system contains the following elements:
- *mRNA encoding the IS30 transposase gene* is *in vitro* transcribed from a plasmid containing the SP6 promoter and serves as source of the transposase when translated in the cell .
- *gfp gene-trap donor* harbors the joined IS30 ends ((*IS30)*_{*2*}) and a marker gene encoding green fluorescent protein (*gfp*). The marker gene is not expected to be expressed in the fish embryo due to the lack of an eukaryotic promoter. The reporter gene was placed downstream of sequences containing a splice-acceptor site of the carp β-actin first intron to create a gene trap construct. Thus, the *gfp* reporter gene can be activated by genomic promoters/enhancers which consequently, provides an *in vivo* assay for monitoring the integration. IS30 transposase requires a circular donor template for the integration reaction. Therefore the donor DNA can be an intact circular plasmid (*GFP plasmid donor)* alternatively, the donor fragment containing the gene trap construct can be excised from the plasmid and religated to generate a circular template *(circular GFP-fg donor).*

### 3.1.1. transpositional target :

Plasmids containing the frequently used integration sites of IS30 (hot spot, Olasz *et al*. 1997a; Olasz *et al* ., 1998) were used. Two types of hot spot sequences were chosen: (i) the consensus sequence of the frequently used integration sites (called *GOHS)* and (ii) the *(IS30)*_{*2*} structures itself. These hot spot sequences were cloned in the first intron (StuI site, nucleotide position +3970) of the *sonic hedgehog (shh)* locus of zebrafish (Chang *et al*., 1997) resulting in the plasmids *GOHS-shh target,* and *(IS30)*_{*2*}*-shh target*. This target plasmid contains the transcriptional regulatory elements of the *shh* gene and therefore, is expected to activate the *gfp* gene of the *gene-trap donor* when the donor sequences are integrated into the first intron of the *shh* gene.

### 3.1.2. Testing of the gene trap insertion system :

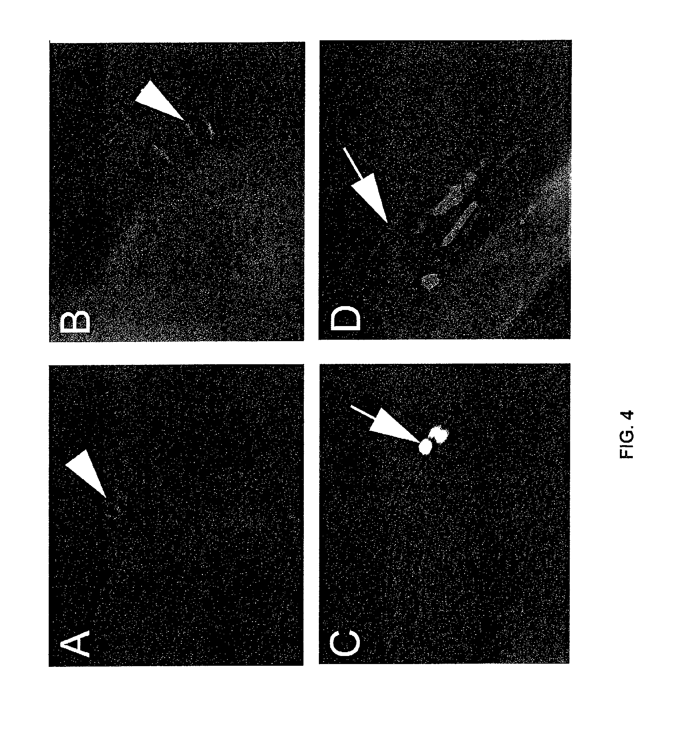

The transpositional target construct was successfully tested in *E. coli* where insertion of the *gfp* donor was detected in the *GOHS* hotspot of the *GOHS-shh target.* The resulting hybrid plasmid containing the gene-trap cassette with the splice-acceptor site and *gfp* marker gene inserted into the first intron of the *shh* locus serves as positive control in zebrafish *(shh-gfp hybrid).* The *shh-gfp* hybrid plasmid was injected into zebrafish embryos and expression of *gfp* was detected at one day of development (Prim. 6 stage), *gfp* activity was found in notochord cells (the cell type which normally expresses the *shh* gene) in 8 out of 30 injected embryos, indicating that the *gfp* marker has been activated under the control of the *shh* regulatory elements (Fig. 4). Therefore, the *gfp* gene inserted into the first intron of the *shh* locus functions as a marker of a *bona fide* gene-trap event. Non-specific activity of *gfp* was also seen due to a background activity of the *gfp* marker probably under the control of cryptic intronic promoter-enhancer elements provided by the carp β-actin intronic sequences adjacent to the splice-acceptor site (Fig. 4C, D).

### 3.2. Insertion of gene trap donor into the shh target plasmid induced by the IS30 transposase in fish embryos

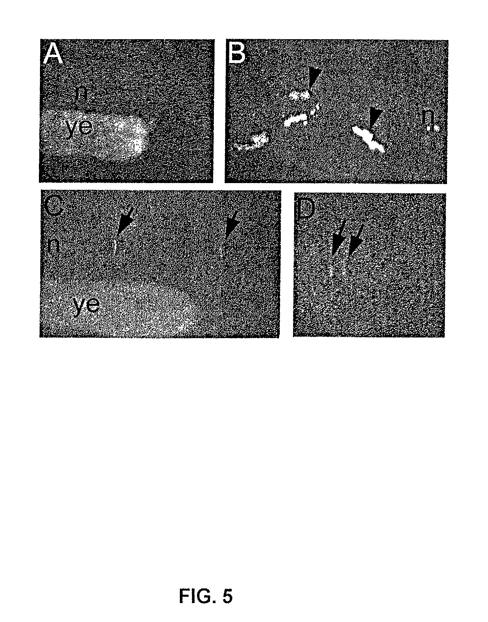

The circular *GFP fragment donor* and the *GOHS-shh target* were injected into zebrafish embryos alone or in combination with synthetic mRNA encoding the IS30 transposase (see schematic in Figure 3). When the IS30 transposase mediates integration of the *gfp-donor* into the *shh-target* in fish embryos, an increase in ventral neural tube and notochord cells expressing *gfp* is expected as a result of a gene trap event. Notochord cells expressing the *gfp* gene were detected in the embryos (4/130 embryos) demonstrating that a gene-trap event in the *shh* locus has been indeed induced by the IS30 transposase (Fig. 5C, D). No expression in the notochord was detected when the target and donor DNA were co-injected without IS30 transposase (Fig. 5B).

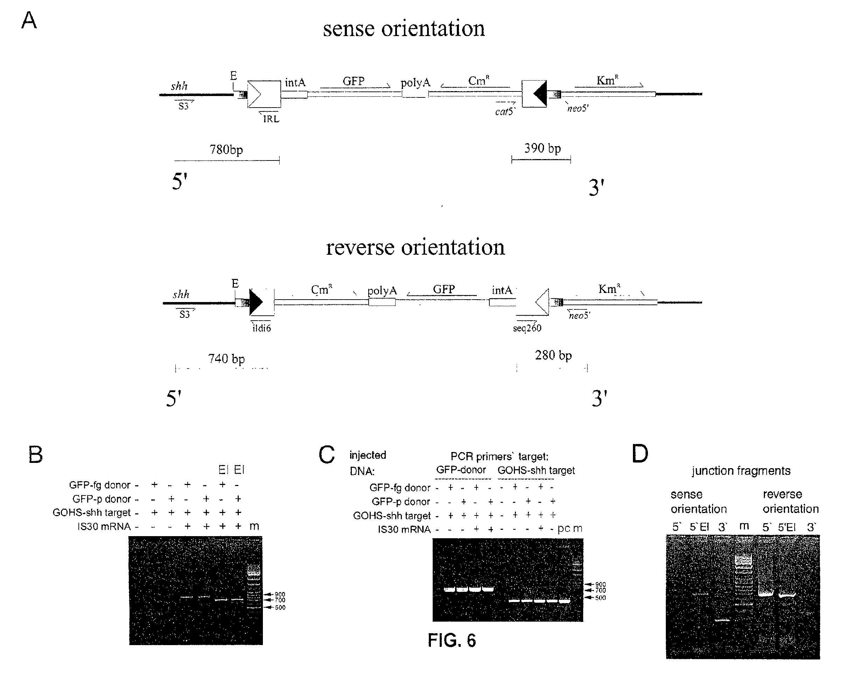

Integration can be detected by PCR amplification of characteristic junction fragments between donor and target sequences. Embryos injected at the one cell stage with combinations of *gfp fragment donor* or the *plasmid GFP donor* and the *GOHS-shh target* plasmid with or without IS30 transposase mRNA were harvested after 10 hours of development and total DNA was prepared. PCR reactions were carried out using the prepared DNA as template and using oligonucleotide primers as shown in Fig. 6A, to detect junction fragments between donor and target plasmids following transposon mediated recombination in the fish embryos. The expected junction fragment indicative of insertion of the circular *GFP-fg donor* or the *plasmid GFP donor* into the *GOHS* hot spot site of the *shh* locus has only been detected in experiments where IS30 mRNA was co-injected, indicating that the transposition was carried out correctly by the transposase (Table V).

**Table V :**

| **Transposition of *gfp* donor DNAs on target plasmids in zebrafish embryos** | | | | |
|---|---|---|---|---|
| | **sense orientation junction fg.** | | **inverse orientation junction fg.** | |
| Construct injected | 5'end | 3' end | 5'end | 3'end |
| 1. circular GFP-fg donor | - | - | - | - |
| 2. plasmid GFP donor | - | - | - | - |
| 3. circular GFP-fg donor + GOHS-*shh* target + IS30 mRNA | + | + | + | + |
| 4. circular GFP-fg donor + GOHS-*shh* target | - | - | - | - |
| 5. plasmid GFP donor + GOHS-*shh* target + IS30 mRNA | + | + | + | + |
| 6. plasmid GFP donor + GOHS-*shh* target | - | - | - | - |

The resulting junction fragments were tested further by restriction digestion confirming the correct identification (Fig. 6B and D). Insertions in both orientations were detected by PCR (Fig. 6D). Expression of *gfp* was monitored in the injected embryos at one day of development. No specific activity was seen in the notochord or floor plate cells, suggesting that the transposition has taken place in a low number of plasmids, below the threshold level required for detectable tissue-specific *gfp* expression. The detection of junction fragments between the target and donor sequences together with the *in vivo* detection of tissue-specifc *gfp* expression demonstrate that transpositional integration generated by the IS30 transposase has taken place in the fish embryos.

### EXAMPLE 4 : SITE-SPECIFIC IS30 INDUCED TRANSPOSITION BY AN IS30 TRANSPOSASE-CI LAMBDA REPRESSOR FUSION PROTEIN IN E. COLI

The efficiency and accuracy of the transposition could be enhanced by modification of transposases. The inventors tested whether the integration frequency could be increased and/or the site of integration can be altered by linking the transposase to a well-defined DNA binding protein. The well-characterised CI repressor of lambda phage was chosen as a targeting protein. A fusion protein (SEQ ID N° 2) was generated containing the CI repressor attached to the IS30 transposase in experiments to define the frequency and target specificity of transposition.

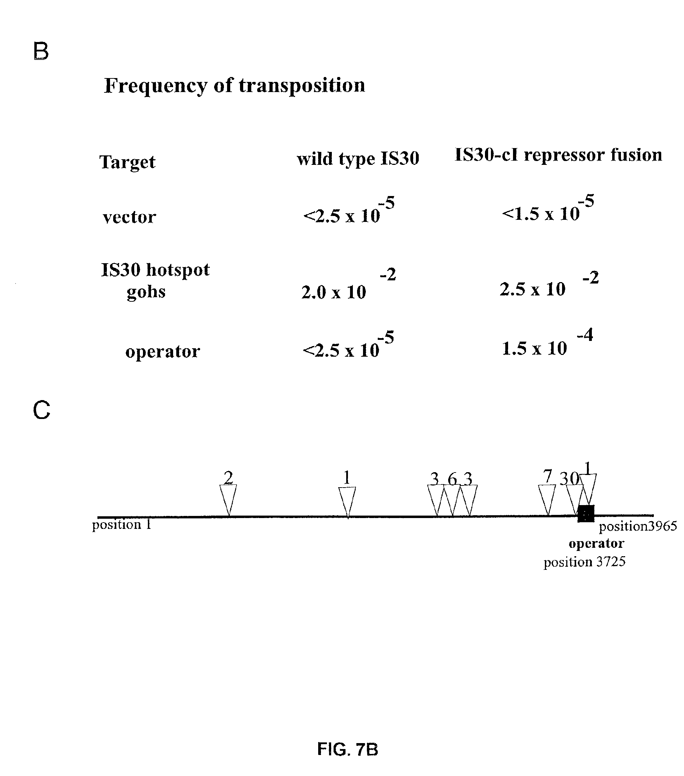

DNAs containing the ORFs of IS30 transposase and that of the lambda CI repressor were PCR amplified, sequenced and fused with a PstI linker. The C-terminal part of the IS30 transposase (1-383 aa) was fused with the corresponding N-terminal part of the full length lambda repressor protein. The two parts of the fusion protein were separated by two unrelated amino acids (Leu, Gln). The ORF was cloned in a *pACYC177* derivative vector (Chang and Cohen, 1978), which contained the *(IS30)*_{*2*} intermediate structure. The inventors have verified that the fused protein retained the activities of both of the transposase and of the CI repressor. Bacteria containing the fusion product were immune against lambda phage infection at 30° C while those containing the IS30 transposase only (without the thermosensitive CI repressor) could be infected by lambda phage. On the other hand, the frequency of transposition by the fusion protein was not affected by the CI fusion as compared to the wild type transposase with the *GOHS* hot target (Fig. 7B).

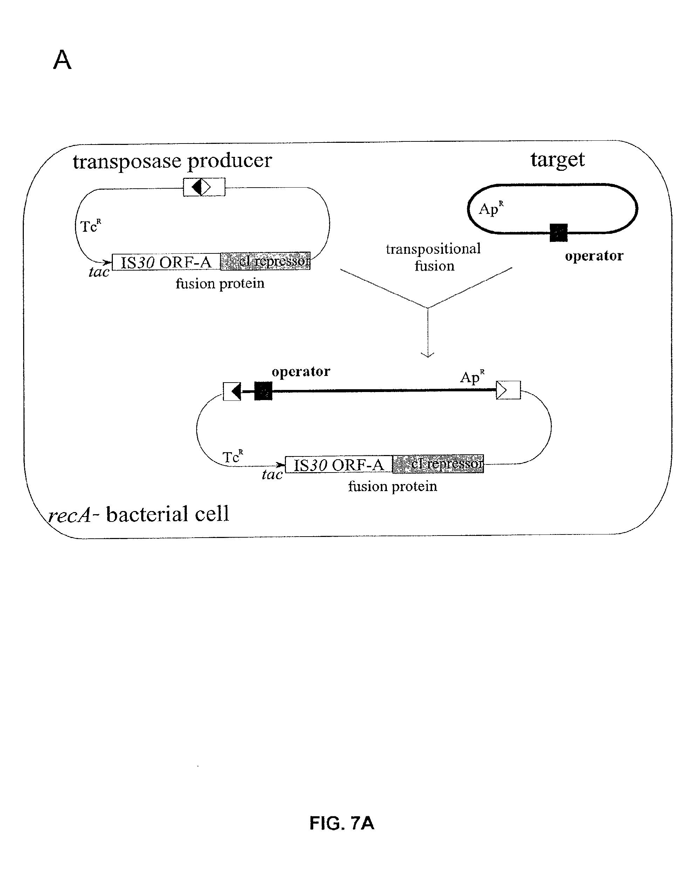

In order to verify that the lambda repressor "domain" in the fusion protein can direct the insertion of the transposable element, the lambda operator region (containing operators OR1, OR2 and OR3) was PCR amplified as an app. 200 bp fragment, cloned and used as target site in a set of experiments (for schematic representation of the experiment see Fig. 7A).

The analysis of the target specificity of insertions mediated by the fusion protein was also performed. The results confirmed that the fusion protein has a directing activity as the majority of insertions were located in the 200-400 bp surroundings of the operator region (Fig. 7C). Moreover, no insertions were found in a control experiment where the same plasmid (*pEMBL19*, Dente *et al*. 1983) but lacking the lambda operator sites was used as target.

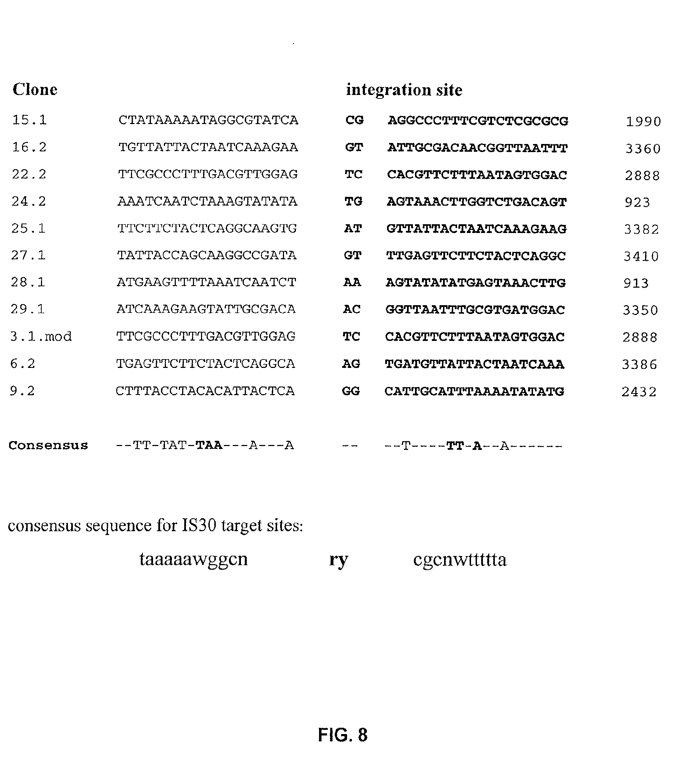

The analysis of the sequences of the insertion sites revealed that only a limited homology was present compared to the consensus sequence for IS30 hot spots (Fig. 8, bold letters in the consensus). There was no significant homology between the insertion sites compared to the consensus sequence of the operator sites of the bacteriophage lambda. These experiments, clearly demonstrate that the IS30 transposase-CI lambda repressor fusion protein efficiently directed transpositional insertions into the proximity of the target.

### EXAMPLE 5 : IDENTIFICATION OF THE TRANSPOSASE DOMAIN RESPONSIBLE FOR TARGET RECOGNITION IN IS30

The *E. coli* resident mobile element IS30 is characterised by a pronounced target specificity (Olasz *et al*., 1998). Upon transposition, the element frequently inserts exactly into the same position of a preferred target sequence. Insertion sites in phages, plasmids and in the genome of *E. coli* are characterised by an exceptionally long palindromic consensus sequence that provides strong specificity for IS*30* insertions despite a relatively high level of degeneracy. This 24 bp long region alone determines the attractiveness of the target DNA and the exact position of IS*30* mediated insertion. This type of target specificity is called: recognition of "natural" hot spots.

It was also verified that the inverted repeats (*IR*) at both ends of the IS30 element also serve as a target for IS30 insertion (Olasz *et al*. 1997a). Not only the single *IRs* but also the *(IS30)*_{*2*} intermediate served as a preferred target site. This kind of target specificity is different to that of "recognition of natural hot spots" and hence called "recognition of *IR* hot spots".

On the basis of this dual target specificity ("natural" and *"IR"* hot spots) it becomes possible to determine the protein domain of the IS30 transposase responsible for the recognition of "natural" target by means of mutational analysis. Namely, mutations in the domain responsible for the recognition of "natural" hot spots have no, or have limited effect on the recognition of *"IR"* hot spots.

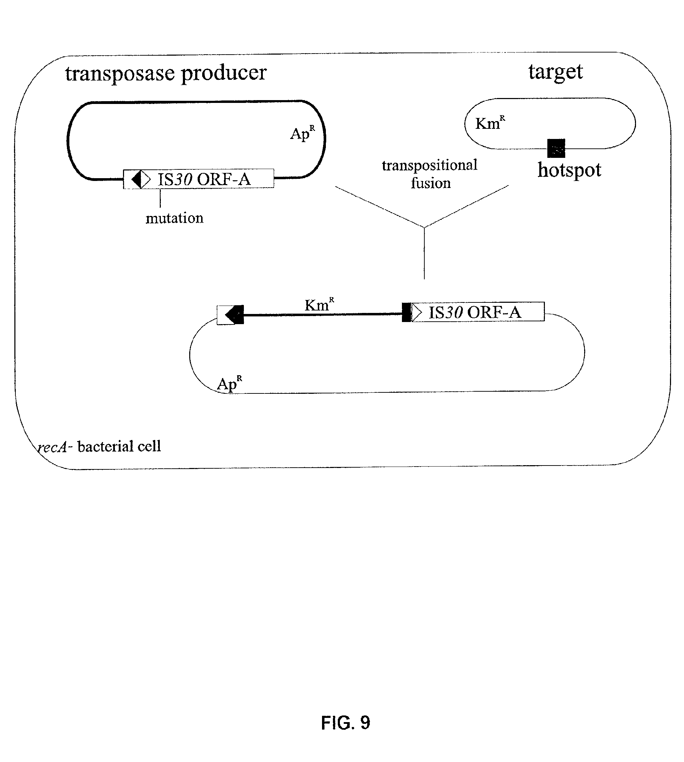

Analysis of the domain structure of the IS30 transposase compared with that of IS30 related elements revealed that there are 3 regions, which could be responsible for the target specificity of the element. Therefore, we focused on the mutational analysis of these regions. One of these regions was located exactly at the beginning of the protein (1-39 aa). The coding region of these amino acids was deleted and the deletion variant transposase was used in a transpositional fusion assay (Fig. 9). The wild type transposase served as a control. Both "natural" *(LHS* and *GOHS)* and *"IR"* (single *IR* and *(IS30)*_{*2*} intermediate) hot spots were offered as target sites (Table VI).

**Table VI :**

| **The N-terminal part of the IS30 transposase is responsible for recognition of the "natural" target sites** | | |
|---|---|---|
| | **Frequency of transposition** | |
| **Offered target** | **Wild type IS30** | **N-terminal deletion of IS30** |
| **natural hot spots** | | |
| *LHS* | 8.0 x 10⁻⁴ | <1.3 x 10⁻⁶ |
| *GOHS* | 1.6 x 10⁻³ | <1.2 x 10⁻⁵ |

| **B, "IR" hot spots** | | |
|---|---|---|
| *IRL -* single IR | 1.7 x 10⁻³ | 1.3 x 10⁻³ |
| *(IS30)*_{*2*} *-* intermediate | 3.5 x 10⁻³ | 1.5 x 10⁻³ |

The deletion of the first 39 amino acids resulted in the loss of recognition of the "natural" hot spot sequences, while other functions of the transposase remained intact (e.g. the recognition of *IR* hot spots); the mutant transposase was still active in transposition (Table V). Therefore it is concluded, that the first 39 amino acids of the IS30 transposase contain the DNA binding domain (DBD) of the transposase responsible for recognition of the target DNA. Based on this result it is possible to exchange the target recognition DBD domain of IS30 with a heterologous DNA-binding domain (e.g. histones, regulatory proteins, recombination enzymes, DNA modifying enzymes etc.). Thus, these experiments clearly show that the development of transposases with altered target specificity is possible.

### EXAMPLE 6 : TARGETED INSERTION BY IS30 IN VERTEBRATE CELLS (FISH EMBRYOS)

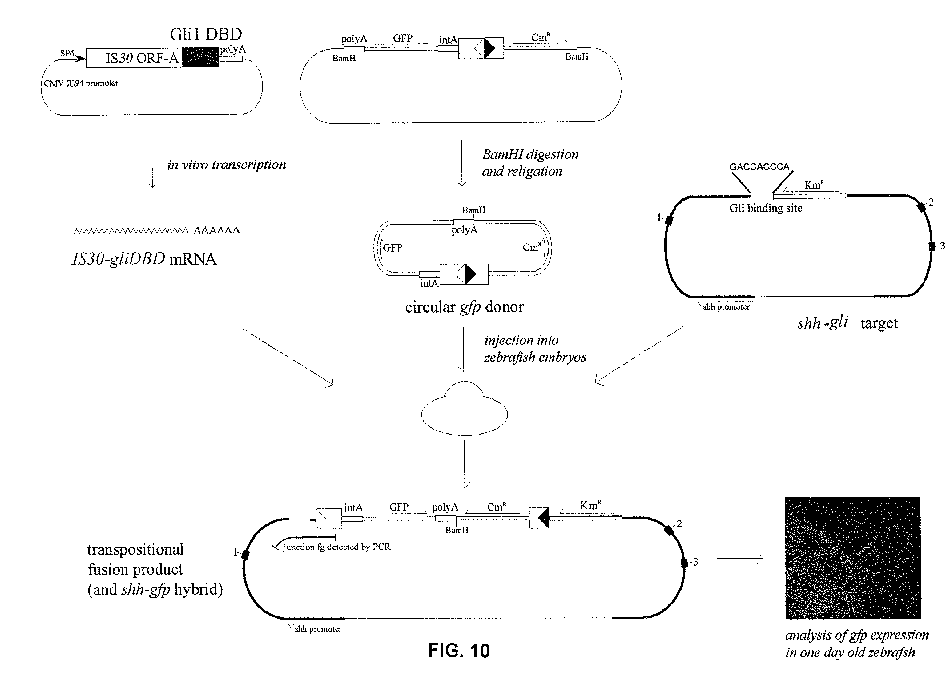

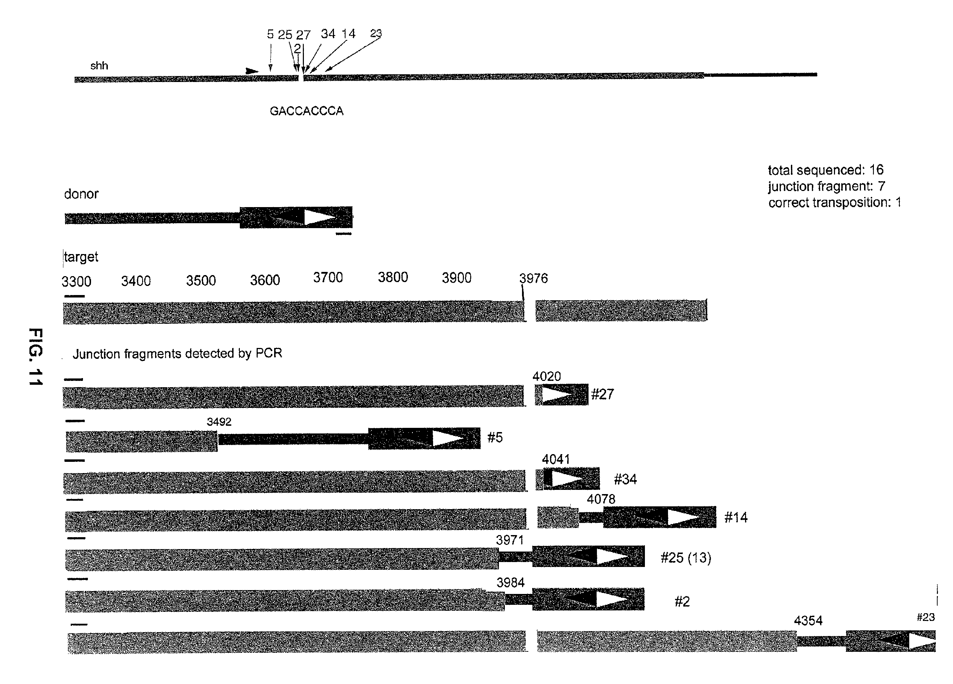

Using a similar strategy to what has been described for the prokaryotic targeting system, a fusion protein was generated and tested for targeting activity in a plasmid system in fish cells. The inventors asked whether an IS30 protein fused to a DNA binding domain would activate insertions in the proximity of a DNA binding recognition sequence on a plasmid system. The DNA binding domain (aa 772 - 1106) of the Gli1 transcription factor of the zinc finger DNA binding protein family (Kinzler *et al*., 1988) was fused to the IS30 coding sequences at the C terminal end of IS30 (SEQ ID N° 4). The resulting fusion protein was expressed in the pCS2+ expression vector. As target DNA for the transposition, a plasmid containing the shh locus was used, with the exception, that the GOHS target hotspot has been replaced by the recognition sequence of Gli1 (GACCACCCA) (Kinzler *et al*., 1990 ; Sasaki *et al*., 1997). As donor DNA the circular gfp fragment was used as described in Fig. 3. The scheme of the targeting experiment in fish is described in Fig. 10. mRNA encoding the *IS30-gliDBD* fusion protein was co-injected into zebrafish 1 cell stage embryos together with gfp circ fg donor and the *shh(gli)* target. As control, injections were carried out without the mRNA or shh target without the Gli binding site. DNA was prepared from gastrula stage embryos and PCR reactions were carried out to detect junction fragments between *shh* target sequences and IS end of donor DNA using primers as demonstrated in Fig. 10 and Fig. 11. The primers S3 and IRL were used to detect insertions in the relative proximity of the Gli1 DNA binding site. This PCR reaction is designed to detect only the insertion of the *gfp* donor fg in the sense orientation. The expected size of the inserted fragment in case of an insertion close to the Glil binding site is expected to be approximately 780 base pairs. PCR reactions using the S3 and IRL oligonucleotides resulted in no amplification in the control injected embryos (target and donor DNAs without *IS30-gliDBD* RNA, or *IS30-gliDBD* mRNA and *gfp* donor fg with target without Gli1 binding site). A smear of bands was detected in the PCR reaction where template DNA was used from embryos injected with all three elements of the transposition system (data not shown). The resulting PCR products were shot gun cloned into a PCR cloning vector (pTOPO, Promega) and a series of plasmids were grown containing different insertion fragments ranging from 500-1500 bps. Clones were picked randomly and sequenced. 16 sequences were obtained representing 16 different clones. In 7 cases a junction fragment between shh and circ gfp fg donor was detected by sequence homology search (bestfit, GCG WISCONSIN PACKAGE, Version 10.2). The junction structure of the 7 clones is demonstrated in Fig. 11. In one case (clone #27) a characteristic junction fragment was detected indicative of a transposition event. The IS dimer structure was resolved at the junction between the *(IS30)*_{*2*}. In this clone, the junction of the IS end to the shh target sequences has occurred at the position 3995, only 36 bases away from the 3' end of the Gli1 DNA binding site, supporting the notion of a targeting event. Further 4 clones contained junction fragments very close to the Gli1 binding site (20-40 bases from the position of G from the GACCACCCA sequence at 3976). In these cases the *(IS30)*_{*2*} dimer was not resolved, suggesting that the insertion took place by illegitimate recombination, and that the fusion protein did not catalyse the resolution of the dimer structure. Since the insertion events were clustered close to the Gli1 DNA binding site, it is argued that the recombination occurred by involvement of binding of the IS30-gli1DBD fusion protein to the Gli1 binding site. It is to be studied further whether the lack of proper transposition is due to steric hindrance of the fusion protein, or other reasons.

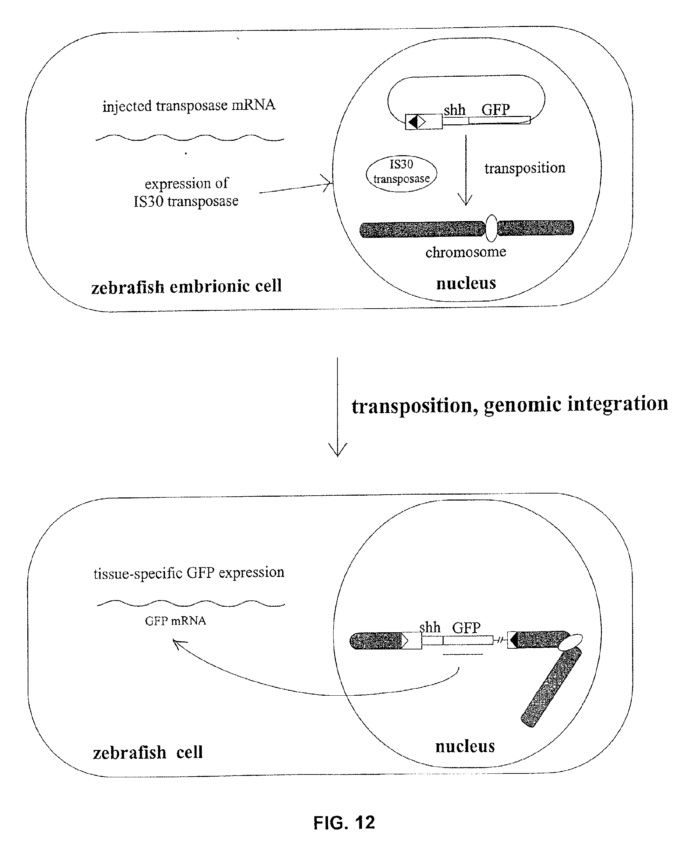

A batch of injected embryos was grown to 24 h and expression of the *gfp* reporter was investigated. Similarly to previous gene trap experiments, expression of the *gfp* donor is expected to be present in the notochord and floor plate cells only in the case of integration on the *shh* sequences in the intronic regions (Fig. 12). The results of the *gfp* expression analysis are shown in Table VII.

**Table VII :**

| **Expression of the donor derived *gfp* gene in notochord cells of zebrafish embryos injected with shh-gli target and the IS30-fliDBD mRNA** | | | |
|---|---|---|---|
| **Treatment** | **total# of embryos** | **# of Gfp+ cells** | **# of Gfp+ Notochord cells** |
| *IS30-Gli1DBD* mRNA+ *gfp fg donor* + *pshh(Gli1)* target | 104 | 161 | 10 |
| | | | |
| *IS30-Gli1DBD* mRNA+ *gfp fg donor+* *pshh* target | 102 | 152 | 0 |
| | | | |
| *gfp fg donor+* *pshh(Gil1)* target | 178 | 262 | 0 |

The above results indicate that the *gfp* donor was expressed in the notochord only in the experimental group where all three elements of the transposition system were injected. This result supports the hypothesis that the IS30-Gli1DBD fusion protein catalyses insertion into the *shh* intronic regions. This result together with the analysis of the junction fragments by PCR provides evidence for the ability of IS30-Gli1DBD to catalyse insertion of donor DNA sequences into the vicinity of a DNA binding site on the donor DNA. Further experiments are required to measure the frequency of the targeting reactions among the total number of recombinations detectable in the plasmid recombination system in the fish embryos.

### EXAMPLE 7 : GENOMIC INTEGRATION OF DNA SEQUENCES INDUCED BY IS30 TRANSPOSITION

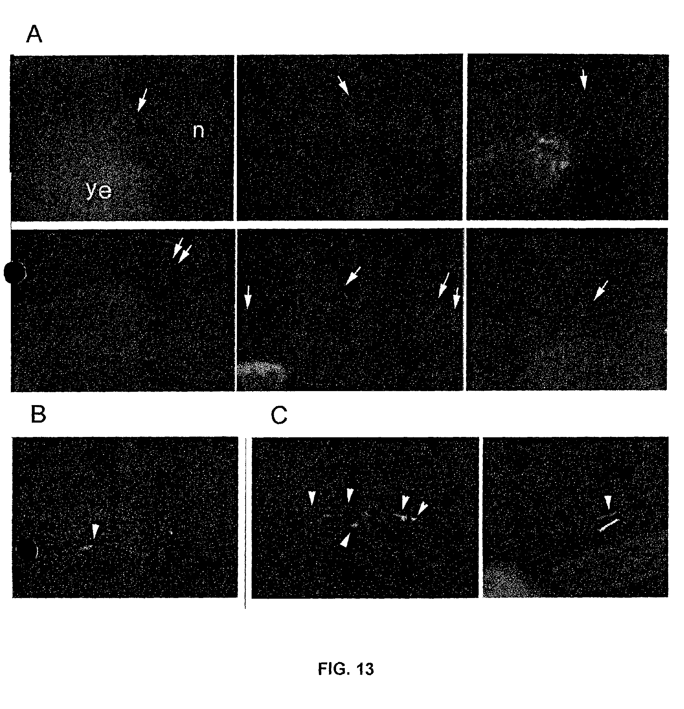

Genomic integration of DNA fragments induced by the IS30 transposase will be tested experimentally as an important element of this invention. It is proposed, that the frequency of genomic integration of transgene constructs can be enhanced by using transgene donor DNA that contains the inverted repeat dimer structure of IS30 *(IS30)*_{*2*} and subsequent co-injection of the donor DNA together with IS30 transposase mRNA in the early embryos (see schematic in Fig. 13). Experiments are under way to detect genomic integration of donor DNA in the zebrafish genome, using a *shh::gfp* expression cassette flanked by the *(IS30)*_{*2*} intermediate structure. The frequency of integration of the expression cassette will be estimated by the frequency of the transmission of the transgene into the F1 generation by the founder P0 parents. F1 embryos will be assayed by DNA detection techniques (PCR, Southern blot hybridization) and by analysis of *gfp* activity. Comparisons will be made between groups injected with the donor construct and IS30 transposase, those injected with the donor construct alone, and those injected with transposase and donor construct not containing the *(IS30)*_{*2*} dimer structure.

### EXAMPLE 8 : REPLACEMENT OF THE TARGET RECOGNITION DOMAIN OF IS30 TRANSPOSASE

The first N-terminal 39 aa of the IS30 transposase was replaced with different parts of the cI repressor of bacteriophage λ - resulting in a hybrid fusion protein. Beside the wild type IS30 transposase, three fusion proteins were constructed, all containing the IS30 transposase domain (40-383 aa) and the DBD domain of the cI repressor but differing in their length :
- ΔDBD-IS30 : IS30 transposase lacking the first 39 aa ;
- HTH-lambdaCI-IS30 : contains only the helix-turn-helix DBD domain of bacteriophage lambda (39 aa) ;
- N-terminal-lambda-IS30 : the first N-terminal 92 aa of the cI repressor was fused to IS30 ;
- Full-lambdaCI-IS30 : the whole cI repressor was fused to the IS30 transposase.

The plasmids used in further experiments contained both the ORF of the fusion proteins and the *(IS30)*_{*2*} intermediate structure, but did not carry any IS30 hotspot or the operator region of bacteriophage λ. The fusion proteins were expressed from the promoter present in the *(IS30)*_{*2*} intermediate structure (Dalrymple, 1987). The activity of the protein was tested in *E. coli* using a deletion assay. In this experimental setup two kinds of target can be used by the IS30 transposase resulting in deletion formation : the IR ends of the IS30 element and heterologous sequences representing the new, gained target specificity of the element. An appropriate *E. coli* strain was transformed with the plasmids selecting for the antibiotic resistance marker of the vector. Single colonies where chosen and were grown in liquid media for 8 generations. Afterwards, plasmid DNA was purified and the structure was analysed with restriction endonucleases.

**Table VIII :**

| **Distribution of the rearrangements mediated by fusion proteins** | | | | |
|---|---|---|---|---|
| **Fusion protein** | **Total tested** | **No rearrangement** | **Target used IR sequence** | **heterologous sequence** |
| wild type IS30 | 29 | 20 | 6 | 3 |
| ΔDBD-IS30 | 20 | 20 | 0 | 0 |
| HTH-lambdaCI-IS30 | 40 | 1 | 22 | 17 |
| N-terminal- lambdacI-IS30 | 40 | 3 | 37 | 0 |
| Full-lambdaCI-IS30 | 40 | 1 | 22 | 17 |

λCI-IS30 fusion proteins were found to be more active than the wild type and the truncated IS30 transposase. Interestingly, the N-terminal-lambdaCI-IS30 fusion protein was only active in the recognition of the IR hot spots, while both the HTH-lambdaCI-1330 and Full-lambdaCI-IS30 fusion also recognised heterologous sequences. These results suggest that the replacement of the first 39 aa of the transposase by a more defined DBD domain can also result in the increase of activity.

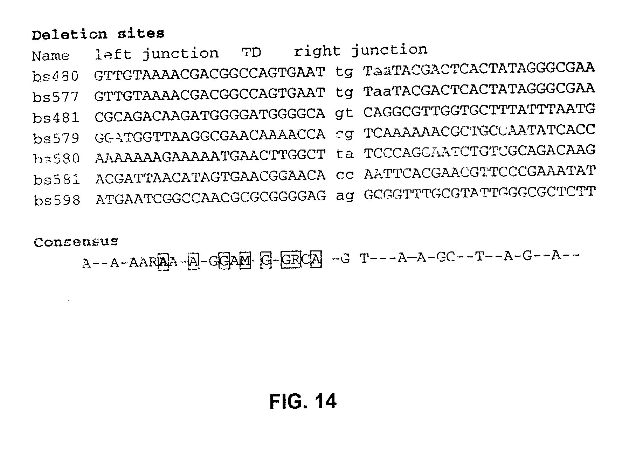

In order to analyse the heterologous sites used in the experiment, the deletion junctions were sequenced (Fig. 14). The sequence analysis confirmed that the fusion protein HTH-lambdaCI-IS30 recognises hot spot sequences, while two sites were used twice (bs480 and bs577). It is important to note, that the consensus sequence derived from 7 independent experiments show significant homology to the lambda operator sequence.

All of these results suggest that the replacement of the target recognition domain of IS30 transposase by a heterologous DBD domain can provide a new specificity to the fusion protein.

### FIGURES

### Figure 1.

Schematic representation of the deletion reaction generated by the IS30 transposase between plasmids in fish embryos. The inverted triangles represent the inverted repeats of the IS30 mobile element that serve as recognition sequences for IS30 on donor constructs. Abbreviations: Ap^{R}, ampicillin resistance, Km^{R} kanamycin resistance, Cm^{R} chloramphenicol resistance, IS30, IS30 transposase ORF.

### Figure 2.

Detection of the mosaically distributed IS30 transposase protein fused to the myc epitope tag by immunohistochemical staining in zebrafish embryos injected with a CMV: IS30 DNA construct. Arrowheads point to cells of the gastrulating zebrafish embryo that contain the fusion protein in their nuclei (upper panel) and in the cytoplasm (lower panel).

### Figure 3.

Schematic representation of the experimental design used to detect transpositional insertion in target plasmids in zebrafish embryos. Abbreviations and symbols are as in Fig. 1 or: sp6, sp6 promoter, intA, carp β-actin gene first intron with splice acceptor, GOHS, *GOHS* hot spot of IS30 transposition, Km^{R} kanamycin resistance gene. Numbers in target and transpositional fusion construct demonstrate the location of the shh exons on the shh locus which is represented by a thick black line.

### Figure 4.

Detection of *gfp* expression in one-day-old zebrafish embryos that contain the *shh-gfp* hybrid construct as described in Fig. 3. The hybrid construct contains the *green fluorescent protein* reporter gene under the control of the *sonic hedgehog* transcriptional regulatory elements resulting in expression of *gfp* in the notochord cells (A,B). Ectopic activity in cells where *shh* is not expressed normally (C, epidermis, D, muscle fibres) has also been detected.

### Figure 5.

Insertion of a *gfp* donor fragment in the shh target locus induced by IS30 transposase results in tissue specific expression of the *gfp* reporter gene in zebrafish embryos. **A,** Tail region of a control non-injected embryo. **B,** Embryo injected with the *circular GFP-fg donor* and *GOHS-shh target* showing non-specific activity in epidermal cells (arrowheads). **C-D,** Embryos injected with *GFP-fg donor* and *GOHS-shh target* together with IS30 transposase express gfp in the notochord cells (arrows) suggesting regulation of the *gfp* gene by the *shh* locus. Abbreviations: n, notochord, ye, yolk extension. Embryos are oriented anterior left except for B where anterior is to the right. Lateral views to the tail region.

### Figure 6.

Detection of junction fragments between the *gfp* donor and *shh* target sequences by PCR.

**A.** Schematic representation of the expected junction fragments amplified by PCR. Arrows below the schematic representation of the DNA indicate oligonucleotidic primers used in the PCR reactions. Expected sizes of PCR products are indicated at the 5' and 3' junctions of recombined DNA fragments. **B.** PCR reactions demonstrating the presence of junction fragments in fish embryos between micro-injected *circular gfp-fg donor* and the *GOHS-shh target* plasmids.

**C.** Control PCR reaction to detect the presence of the original donor and target molecules in DNA purified from micro-injected fish embryos. **D.** PCR reaction to detect the junction fragments produced by recombination of the *circular gfp-fg donor* and *GOHS shh-target* in different orientations. Abbreviations: GFP-fg: *circular grp-fg donor,* GFP-p: *gfp plasmid donor*, GOHS-shh: *GOHS-shh target* plasmid, EI: Eco RI digested PCR product, m: molecular weight marker, pc: *shh-gfp* hybrid plasmid control, 5': junction fragment generated at the 5' end of inserted donor, 3': junction fragment generated at the 3' end of inserted donor.

### Figure 7.

**A.** Schematic representation of the transposition system based on transposase fusion proteins. **B.** Results of transposition experiments using different target sequences and fusion variants of IS30. **C.** The insertion sites of the transposase producer donor in the target plasmid. Numbers and triangles represent sites and number of integration events detected in these particular sites. Abbreviations as before, and: tac: tac promoter, TcR, tetracycline resistance gene.

### Figure 8.

Analysis of the insertions mediated by the IS30 transposase-CI lambda repressor fusion protein. On the right panel, the position of integration is presented according to the *pEMBL19* sequence. The site of target duplication in the sequences (2 bp in the middle) are separated from the flanking regions by space. Bold letters in the consensus represent matches to the CIG consensus sequence for IS30 target sites (Olasz et *al.* 1998), w, r and y in the consensus represent A or T, purin and pyrimidine bases, respectively.

### Figure 9.

Schematic representation of the transpositional integraton system used to test the efficiency of a deletion variant of IS30. Abbreviations as before.

### Figure 10.

Schematic representation of the transposition system used to detect targeted transposition between plasmids in fish embryos. Abbreviations and symbols as in Fig. 4 with the following exceptions : yellow bar in *shh-(Gli1)* target and *shh-gfp* hybrid fusion product represents the insertion of a GACCACCCA sequence starting in the position 3976 of the *shh* genomic locus in intron I. Arrows represent annealing positions of S3 and IRL oligonucleotides used in PCR reactions to detect junction fragments. Red bar represents aa 772 to 1106 of the mouse Glil1 zinc finger domains fused to the IS30 transposase.

### Figure 11.

Analysis of the fusion fragments detected by PCR between *shh* and IS end of *gfp* donor sequences. Red bars with triangles represent IS end sequences, green bars represent *shh-gli* donor sequences. Yellow bars represent the Gli1 DNA binding site (GACCACCCA sequence). Numbers above the junction fragments demonstrate the position of integration of IS end containing donor sequences to the *shh-gli* target DNA. Black bars demonstrate the positions where S3 and IRL oligonucleotides anneal to target sequences in the PCR reactions.

### Figure 12.

Expression of *gfp* was detected in embryos injected with all three components of the IS30 transposition system. Experimental conditions and abbreviations are as described in Fig. 4. Details of *gfp* expression analysis are described in Table VI.

### Figure 13.

Schematic representation of the transpositional integration system used in zebrafish to enhance transgene integration efficiency. Abbreviations: shh, shh promoter and cis regulatory elements. GFP, *gfp* gene.

### Figure 14.

Analysis of the deletions mediated by the IS30 transposase-CI lambda repressor fusion protein. The site of target duplication in the sequences (2 bp in the middle - TD) are separated from the flanking regions by a space. Boxes in the consensus represent matches to the lambda operator. R and M in the consensus represent A or T, A or C bases, respectively.

### REFERENCES

Bell *et al*. (1999) Exp. Cell. Res. **246:**11-19.
Chang *et al.* (1978) J. Bact. **134:**1141-1156.
Chang *et al.* (1997) EMBO J. **16:**3955-3964.
Dalrymple *et al.* (1984) EMBO J. **3:**2145-2149.
Dalrymple *et al.* (1987) Mol. Gen. Genet. **207:**413-420.
Dente *et al.* (1983) EMBL Nucleic Acids Res. **11:** 1645-1655.
Farkas *et al*. (1996) FEBS Letters **390:**53-58.
Gossler *et al.* (1989) Science, **244:**463-465.
Ivics *et al.* (1997) Cell **91:**501-510.
Izsvak *et al.* (1995) Mol. Gen. Genet. **247:**312-22.
Jenuwein (2001) Trends Cell. Biol. **11:**266-273.
Kawakami *et al.* (2000) Proc. Natl. Acad. Sci. USA **97:**11403-11408.
Kim *et al*. (2000) Mol. Cells **10:**367-374.
Kinzler *et al.* (1988) Nature **332:**371-374.
Kinzler *et al.* (1990) Mol. Cell. Biol. **10:**634-642.
Kiss *et al*. (1999) Molec. Microbiol. **34:**37-52.
Lam *et al.* (1996) Proc. Natl. Acad. Sci. USA **93:**10870-10875.
Mahillon *et al.* (1998) Microbiol. Mol. Biol. Rev. **62:**725-774.
Malik *et al.* (2000) Trends Biochem. Sci. **25:**277-283.
Marmorstein *et al.* (2001) Curr. Opin. Genet. Dev. **11:**155-161.
Muller *et al*. (1999) Development **126:**2103-2116.
Olasz *et al.* (1993) Mol. Gen. Genet. **239:**177-187.
Olasz *et al.* (1997a) J. Bacteriol. **179:**7551-7558.
Olasz *et al.* (1997b) FEBS Letters **413:**453-461.
Olasz *et al.* (1998) Mol. Microbiol. **28:**691-704.
Ploy *et al.* (2000) Clin. Chem. Lab. Med. **38:**483-487.
Raz *et al.* (1998) Curr. Biol. **8:**82-88.
Sambrook *et al.* (1989) Cold Spring Harbor Laboratory Press NY, USA.
Sasaki *et al*. (1997) Development **124:**1313-1322.
Skarnes (1990) Biotechnology **8:**827-831.
Stark *et al.* (1989) Trends Genet. **5:**304-309.
Turner *et al.* (1994) Genes Dev. **8**:1434-1447.

## Claims

1. A fusion protein comprising at least : (i) a site-directed recombinase protein, or a fragment or a variant thereof and, (ii) a heterologous protein, or a fragment or a variant thereof, that binds either directly or indirectly DNA at least at one responsive element (RE), said fusion protein having a heterologous site-directed recombinase activity such that in a cell or cell free system containing said responsive element, said fusion protein binds directly or indirectly to said responsive element and catalyzes recombination in the vicinity or at said DNA responsive element in presence of site-directed recombinase targeting sequence (SDRTS).

2. Fusion protein according to claim 1 wherein the endogenous DNA-binding domain of said site-directed recombinase protein or a fragment or a variant thereof, is not functional such that said fusion protein does not catalyze recombination at the natural endogenous targeting sequence of said site-directed recombinase protein.

3. Fusion protein according to claims 1 and 2 wherein said site-directed recombinase is selected among prokaryotic site-directed recombinases and eukaryotic site-directed recombinases.

4. Fusion protein according to claim 3 wherein said site-directed recombinases are selected in the group comprising transposases and DNA recombinases.

5. Fusion protein according to claim 4 wherein said transposase is selected from the transposases encoded by sequences derived from a transposable element selected from the group consisting of:
- transposons Tn3, Tn5, Tn7, Tn10, Tn916; and
- procaryotic mobile elements IS1, IS2, IS3, IS5, IS10L, IS10L/R-2, IS26, IS30, IS50, IS150, IS186, IS911, and
- eukaryotic mobile elements Tc1 of *Caenorhabditis elegans*, the P and Copia elements of *Drosophila,* the synthetic element Sleeping Beauty, Hobo, Mariner, Ac-Ds Spm, the Mu elements of maize, Ty1, Ty2, Ty3 elements of yeast, Tam1, Tam2 and Tam3 elements of *Antirrhinum,* Tx1 and Tx2 elements of *Xenopus;* and
- retrotransposons such as blood, gypsy, springer and beagle elements of *Drosophila;* and
- integrases of retroviruses RSV, SNV, Mo-MLV, MMTV, HTLV1 and HIV1;
and their fragments or variants thereof.

6. Fusion protein according to claim 5 wherein said transposase is selected from the transposases able to form covalently joined SDRTS sequences such as sequences derived from procaryotic mobile elements IS1, IS2, IS3, IS5, IS10L, IS10L/R-2, IS26, IS30, IS50, IS150, IS186, IS911.

7. Fusion protein according to claim 6 wherein said transposase is IS30 transposase from *Escherichia coli*.

8. Fusion protein according to claim 5 wherein said transposase is selected from the transposases able to form covalently joined SDRTS sequences such as eukaryotic mobile elements Tc1 of *Caenorhabditis elegans,* the P and Copia elements of *Drosophila,* Ac-Ds Spm and Mu elements of maize, Ty1, Ty2, Ty3 elements of yeast, Tam1, Tam2 and Tam3 elements of *Antirrhinum,* Tx1 and Tx2 elements of *Xenopus;*

9. Fusion protein according to claim 4 wherein said DNA recombinase is selected from the group of site-directed recombinases comprising the Cre recombinase of bacteriophage P1, the FLP recombinase of *Saccharomyces cerevisiae,* the R recombinase of *Zygosaccharomyces rouxii* pSR1, the A recombinase of *Kluyveromyces drosophilarium* pKD1, the A recombinase of *Kluyveromyces waltii* pKW1, the integrase λ Int, the recombinase of the GIN recombination system of the Mu bacteriophage, the recombinase of the CIN recombination system of P1 bacteriophage, the recombinase of the MIN recombination system of P1-15 bacteriophage, the bacterial β recombinase, the invertase of the Hin system of *Salmonella,* the invertase of the FIM system of *Escherichia*, the integrase of the SLP1 system of *Streptomyces* or variants thereof.

10. Fusion protein according to claims 1 to 9 wherein said heterologous protein, or a fragment or a variant thereof comprises at least a DNA binding domain, said heterologous protein being selected among site-specific recombinases, transposases, procaryotic host factors, procaryotic activator proteins, procaryotic repressor proteins, eukaryotic transcription factors, DNA methylases, restriction endonucleases, chromatin proteins, or a fragment or a variant thereof.

11. Fusion protein according to claim 10 wherein said heterologous protein is the procaryotic repressor protein cI of the lambda phage, and the variants thereof.

12. Fusion protein according to claim 10 wherein said heterologous protein is the eukaryotic zinc-finger transcription factor Gli, and the variants thereof.

13. Fusion protein according to claim 10 wherein said heterologous protein is the Tet repressor and its variants thereof encoded by the tetracycline resistance gene.

14. Fusion protein according to claims 1 to 9 wherein said heterologous protein, or a fragment or a variant thereof that binds directly or indirectly DNA is selected among proteins that are known to associate with DNA binding factor.

15. Fusion protein according to claims 1 to 14 wherein said DNA responsive element is selected among operator region of procaryotic repressors, methylation sites of sequence-specific methylases, recognition sites of restriction endonucleases, binding sites of host factors, recognition sequence of site-specific recombinases, hot spot sequences for transposases, transcription factors responsive elements, the phage λ operator region, the CpG island, LHS, GOHS, IS30 recognition sequence, (*IS30)*_{*2*}.

16. Fusion protein according to claim 15 wherein said DNA responsive element is the operator region of phage λcI repressor.

17. Fusion protein according to claims 1 and 2 wherein said heterologous protein is the cI repressor of the lambda phage or a fragment or a variant thereof and said transposase is IS30 transposase from *Escherichia coli* or a fragment or a variant thereof.

18. Fusion protein according to claim 17 of sequence SEQ ID N° 2.

19. Fusion protein according to claims 1 and 2 wherein said heterologous protein is the DNA-binding domain of Gli transcription factor or a fragment or a variant thereof and said transposase is IS30 transposase from *Escherichia coli* or a fragment or a variant thereof.

20. Fusion protein according to claims 19 of sequence SEQ ID N° 4.

21. Fusion protein according to claims 1 and 2 wherein said heterologous protein is Tet repressor or a fragment or a variant thereof and said transposase is IS30 transposase from *Escherichia coli* or a fragment or a variant thereof.

22. Fusion protein according to claim 21 of sequence SEQ ID N° 6.

23. Recombinant polynucleotide encoding for a protein fusion according to claims 1 to 22.

24. Recombinant polynucleotide of sequence SEQ ID N° 1 encoding for the fusion protein according to claim 18.

25. Recombinant polynucleotide of sequence SEQ ID N° 3 encoding for the fusion protein according to claim 20.

26. Recombinant polynucleotide of sequence SEQ ID N° 5 encoding for the fusion protein according to claim 22.

27. Recombinant polynucleotide comprising a polynucleotide selected among :
a) The polynucleotide according to claims 23 to 26 ;
b) The polynucleotides presenting at least 70% identity after optimal alignment with the polynucleotide of step a) ;
c) Fragments of polynucleotides of claims 23 to 26 encoding for a peptide having at least a site-directed recombinase activity;
d) The complementary sequence or RNA sequence corresponding to a polynucleotide of step a), b) or c).

28. A DNA cassette comprising a promoter operably linked to a polynucleotide according to claims 23 to 27.

29. Vector comprising a polynucleotide according to claims 23 to 27.

30. Expression vector comprising at least one DNA cassette according to claim 28.

31. Gene targeting vector comprising at least:
a) A gene encoding a fusion protein according to claims 1 to 22 or a polynucleotide according to claims 23 to 27 ;
b) Optionally, a promoter that is operatively linked to said fusion protein gene or to said polynucleotide;
c) Optionally, a DNA responsive element recognized by the DNA binding domain of said fusion protein ;
d) At least, two SDRTS recognized by the recombinase domain of said fusion protein ;
e) At least, one transposable DNA sequence of interest, wherein said DNA sequence of interest is located between said two SDRTS sequences ;
f) Optionally, at least one marker gene;
and wherein one of said SDRTS is located between said fusion protein encoding gene or said polynucleotide and said DNA sequence of interest.

32. Gene targeting vector comprising at least:
a) A gene encoding a fusion protein according to claims 1 to 22 or a polynucleotide according to claims 23 to 27 ;
b) Optionally a promoter that is operatively linked to said fusion protein gene or to said polynucleotide;
c) Optionally, one DNA responsive element recognized by the DNA binding domain of said fusion protein ;
d) At least two SDRTS recognized by the recombinase domain of said fusion protein, said SDRTS being joined covalently together and being separated at most by 20bp;
e) Optionally, at least one marker gene.

33. Gene targeting vector comprising:
a) Optionally, a DNA responsive element recognized by the DNA binding domain of a fusion protein according to claims 1 to 22 ;
b) At least, two SDRTS recognized by the recombinase domain of said fusion protein according to claims 1 to 22 ;
c) At least, one transposable DNA sequence of interest, wherein said gene is located between said two SDRTS sequences ;
d) Optionally, at least one marker gene.

34. Vector according to claims 30 to 33 wherein the said promoter is inducible by an inducing stimulus to cause the transcription of said fusion protein encoding gene or said polynucleotide.

35. A vector according to claims 29 to 34 whereby said vector is derived from a viral vector, preferably an adenoviral, retroviral, or adeno-associated viral vector.

36. A kit to perform site-directed recombination wherein said kit comprises at least :
(i) a gene encoding a fusion protein according to claims 1 to 22 or a polynucleotide according to claims 23 to 27, optionally a promoter that is operatively linked to said fusion protein gene or to said polynucleotide, or a DNA cassette according to claim 28, or a vector according to one of claims 29 or 30 ; and
(ii) a donor DNA molecule that comprises a transposable DNA sequence of interest, said DNA sequence of interest being flanked at its 5' and/or 3' ends by said SDRTS, said SDRTS being specifically recognized by the recombinase domain of the fusion protein encoded by a gene, a polynucleotide, a DNA cassette, a vector of step (i) ;
(iii) Optionally, a recipient DNA molecule that comprises at least one DNA responsive element that binds the DNA-binding domain of the fusion protein encoded by a gene, a polynucleotide, a DNA cassette or a vector of (i).

37. A kit to perform site-directed recombination wherein said kit comprises at least :
(i) a fusion protein according to claims 1 to 22 ; and
(ii) a donor DNA molecule that comprises a transposable DNA sequence of interest, said DNA sequence of interest being flanked at its 5' and/or 3' ends by said SDRTS, said SDRTS being specifically recognized by the recombinase domain of the fusion protein of (i) ;
(iii) optionally, a recipient DNA molecule that comprises at least one DNA responsive element that binds the DNA-binding domain of the fusion protein of (i).

38. A kit to perform site-directed recombination wherein said kit comprises at least :
(i) a gene targeting vector according to claims 31 to 33 ; and
(ii) optionally, a recipient DNA molecule that comprises at least one DNA responsive element that binds the DNA-binding domain of the fusion protein of (i).

39. A host cell transformed by at least one polynucleotide according to claims 23 to 27, one DNA cassette according to claim 28, one vector according to claims 29 to 35.

40. Cell according to claim 39 wherein the protein fusion encoded by said polynucleotide according to claims 23 to 27, DNA cassette according to claim 28, or vector according to claims 29 to 35, is expressed and biologically active in said cell.

41. Cell according to one of claims 39 or 40 wherein said cell is an eukaryotic cell selected among human cells, murine cells, yeast cells, amphibian cells, fish cells, drosophila cells, *Caenorhabditis* cells, plant cells.

42. Cell according to one of claims 39 or 40 wherein said cell is a procaryotic cell selected among *Escherichia sp., Bacillus sp., Campylobacter sp., Helicobacter sp., Agrobacterium sp., Staphylococcus sp., Thermophilus sp., Azorhizobium sp., Rhizobium sp., Neisseria sp., Neisseria sp., Pseudomonas sp., Mycobacterium sp., Streptomyces sp., Corynebacterium sp., Lactobacillus sp., Micrococcus sp., Yersinia sp*., *Brucella sp., Bortadella sp., Proteus sp., Klebsiella sp., Erwinia sp., Vibrio sp., Photorhabdus sp., desulfovibrio sp., Listeria sp., Clostridium sp., Actynomyces sp., Haemophilus sp..*

43. Animal, excepted humans, comprising at least a cell according to claims 39 to 41.

44. Animal according to claim 43 wherein said animal is a mouse.

45. A method for *in vitro* site-directed DNA recombination, said method comprising the steps of combining :
a) a donor DNA molecule that comprises a transposable DNA sequence of interest, the DNA sequence of interest being flanked at its 5' and/or 3' ends by said SDRTS being specifically recognized by the recombinase domain of the fusion protein according to claims 1 to 22 ; with
b) a recipient DNA molecule that comprises at least one DNA responsive element that binds the DNA-binding domain of the fusion protein according to claims 1 to 22 ; with
c) a fusion protein according to claims 1 to 22.

46. A method for *in vivo* site-directed DNA recombination, said method comprising the steps of combining into a cell:
a) A donor DNA molecule that comprises a transposable DNA sequence of interest, the DNA sequence of interest being flanked at its 5' and/or 3' ends by SDRTS being specifically recognized by the recombinase domain of the fusion protein according to claims 1 to 22 ; with
b) A recipient DNA molecule that comprises at least one DNA responsive element that binds the DNA-binding domain of the fusion protein according to claims 1 to 22 ; with
c) A fusion protein according to claims 1 to 22 or a gene encoding a fusion protein according to claims 1 to 22 or a polynucleotide according to claims 23 to 27, optionally a promoter that is operatively linked to said fusion protein gene or to said polynucleotide, or a DNA cassette according to claim 28, or a vector according to claims 29 and 30, said gene or polynucleotide being efficiently expressed in said cell.

47. A method according to one of claims 45 or 46 wherein the DNA recombination is selected among insertion, deletion, inversion, translocation, fusion.

48. A method for the stable introduction of a DNA sequence of interest into at least one recipient DNA molecule of a cell, said recipient DNA molecule comprises at least one DNA responsive element that binds the DNA-binding domain of the fusion protein according to claims 1 to 22, and said method comprising :
a) providing a donor DNA molecule that comprises a transposable DNA sequence of interest, the DNA sequence of interest being flanked at its 5' and/or 3' ends by said SDRTS being specifically recognized by the recombinase domain of the fusion protein according to claims 1 to 22 ;
b) introducing into the cell said donor DNA molecule; and,
c) previously, simultaneously, or separately, introducing into said cell a fusion protein according to claims 1 to 21 or a gene encoding a fusion protein according to claims 1 to 22 or a polynucleotide according to claims 23 to 27, optionally a promoter that is operatively linked to said fusion protein gene or to said polynucleotide, or a DNA cassette according to claim 28, or a vector according to one of claims 29 or 30, said gene or polynucleotide being efficiently expressed in said cell.

49. Use of a vector according to claims 31 to 33 as a functional transposon in integrating a nucleic acid sequence of interest into a genome of a cell.

50. Vector according to claims 29 to 35 as a medicament.

51. Use of a vector according to claim 50 to perform a gene therapy to a patient in need of such treatment.

52. Use of a fusion protein according to claims 1 to 22, or a polynucleotide according to claims 23 to 27, or a vector according to claims 29 to 35, or a DNA cassette according to claim 28, or a kit according to claims 36 and 38, for producing transgenic cells and/or transgenic animals.

53. Use of a fusion protein according to claims 1 to 22, or a polynucleotide according to claims 23 to 27, or a vector according to claims 29 to 35, or a DNA cassette according to claim 28, or a kit according to claims 36 and 38 to perform gene targeting, gene knock-out (KO), gene knock-in (KI), gene-trapping, transposon tagging.


SEQUENCE LISTING

<110> Association pour le développement de la recherche en génétique moléculaire
- ADEREGEM

<120> Site-directed recombinase fusion proteins and corresponding
polynucleotides, vectors and kits, and their uses for site-directed DNA
recombination

<130> D19376

<160> 6

<170> PatentIn version 3.0

<210> 1
<211> 1937
<212> DNA
<213> Artificial sequence

<220>
<223> Is30-ci repressor fusion protein

<220>
<221> CDS
<222> (63)..(1934)

<400> 1
tgtagattca attggtcaac gcaacagtta tgtgaaaaca tggggttgcg gaggtttttt 60

ga atg aga cga aca ttt aca gca gag gaa aaa gcc tct gtt ttt gaa 107
Met Arg Arg Thr Phe Thr Ala Glu Glu Lys Ala Ser Val Phe Glu
1               5                   10                  15

cta tgg aag aac gga aca ggc ttc agt gaa ata gcg aat atc ctg ggt 155
Leu Trp Lys Asn Gly Thr Gly Phe Ser Glu Ile Ala Asn Ile Leu Gly
                20                  25                  30

tca aaa ccc gga acg atc ttc act atg tta agg gat act ggc ggc ata 203
Ser Lys Pro Gly Thr Ile Phe Thr Met Leu Arg Asp Thr Gly Gly Ile
            35                  40                  45

aaa ccc cat gag cgt aag cgg gct gta gct cac ctg aca ctg tct gag 251
Lys Pro His Glu Arg Lys Arg Ala Val Ala His Leu Thr Leu Ser Glu
        50                  55                  60

cgc gag gag ata cga gct ggt ttg tca gcc aaa atg agc att cgt gcg 299
Arg Glu Glu Ile Arg Ala Gly Leu Ser Ala Lys Met Ser Ile Arg Ala
    65                  70                  75

ata gct act gcg ctg aat cgc agt cct tcg acg atc tca cgt gaa gtt 347
Ile Ala Thr Ala Leu Asn Arg Ser Pro Ser Thr Ile Ser Arg Glu Val
80                  85                  90                  95

cag cgt aat cgg ggc aga cgc tat tac aaa gct gtt gat gct aat aac 395
Gln Arg Asn Arg Gly Arg Arg Tyr Tyr Lys Ala Val Asp Ala Asn Asn
                100                 105                 110

cga gcc aac aga atg gcg aaa agg cca aaa ccg tgc tta ctg gat caa 443
Arg Ala Asn Arg Met Ala Lys Arg Pro Lys Pro Cys Leu Leu Asp Gln

            115                 120                 125

aat tta cca ttg cga aag ctt gtt ctg gaa aag ctg gag atg aaa tgg 491
Asn Leu Pro Leu Arg Lys Leu Val Leu Glu Lys Leu Glu Met Lys Trp
        130                 135                 140

tct cca gag caa ata tca gga tgg tta agg cga aca aaa cca cgt caa 539
Ser Pro Glu Gln Ile Ser Gly Trp Leu Arg Arg Thr Lys Pro Arg Gln
    145                 150                 155

aaa acg ctg cga ata tca cct gag aca att tat aaa acg ctg tac ttt 587
Lys Thr Leu Arg Ile Ser Pro Glu Thr Ile Tyr Lys Thr Leu Tyr Phe
160                 165                 170                 175

cgt agc cgt gaa gcg cta cac cac ctg aat ata cag cat ctg cga cgg 635
Arg Ser Arg Glu Ala Leu His His Leu Asn Ile Gln His Leu Arg Arg
                180                 185                 190

tcg cat agc ctt cgc cat ggc agg cgt cat acc cgc aaa ggc gaa aga 683
Ser His Ser Leu Arg His Gly Arg Arg His Thr Arg Lys Gly Glu Arg
            195                 200                 205

ggt acg att aac ata gtg aac gga aca cca att cac gaa cgt tcc cga 731
Gly Thr Ile Asn Ile Val Asn Gly Thr Pro Ile His Glu Arg Ser Arg
        210                 215                 220

aat atc gat aac aga cgc tct cta ggg cat tgg gag ggc gat tta gtc 779
Asn Ile Asp Asn Arg Arg Ser Leu Gly His Trp Glu Gly Asp Leu Val
    225                 230                 235

tca ggt aca aaa aac tct cat ata gcc aca ctt gta gac cga aaa tca 827
Ser Gly Thr Lys Asn Ser His Ile Ala Thr Leu Val Asp Arg Lys Ser
240                 245                 250                 255

cgt tat acg atc atc ctt aga ctc agg ggc aaa gat tct gtc tca gta 875
Arg Tyr Thr Ile Ile Leu Arg Leu Arg Gly Lys Asp Ser Val Ser Val
                260                 265                 270

aat cag gct ctt acc gac aaa ttc ctg agt tta ccg tca gaa ctc aga 923
Asn Gln Ala Leu Thr Asp Lys Phe Leu Ser Leu Pro Ser Glu Leu Arg
            275                 280                 285

aaa tca ctg aca tgg gac aga gga atg gaa ctg gcc aga cat cta gaa 971
Lys Ser Leu Thr Trp Asp Arg Gly Met Glu Leu Ala Arg His Leu Glu
        290                 295                 300

ttt act gtc agc acc ggc gtt aaa gtt tac ttc tgc gat cct cag agt 1019
Phe Thr Val Ser Thr Gly Val Lys Val Tyr Phe Cys Asp Pro Gln Ser
    305                 310                 315

cct tgg cag cgg gga aca aat gag aac aca aat ggg cta att cgg cag 1067
Pro Trp Gln Arg Gly Thr Asn Glu Asn Thr Asn Gly Leu Ile Arg Gln
320                 325                 330                 335

tac ttt cct aaa aag aca tgt ctt gcc caa tat act caa cat gaa cta 1115
Tyr Phe Pro Lys Lys Thr Cys Leu Ala Gln Tyr Thr Gln His Glu Leu
                340                 345                 350

gat ctg gtt gct gct cag cta aac aac aga ccg aga aag aca ctg aag 1163
Asp Leu Val Ala Ala Gln Leu Asn Asn Arg Pro Arg Lys Thr Leu Lys
            355                 360                 365

ttc aaa aca ccg aaa gag ata att gaa agg ggt gtt gca ttg aca gat 1211
Phe Lys Thr Pro Lys Glu Ile Ile Glu Arg Gly Val Ala Leu Thr Asp
        370                 375                 380

ctg cag atg agc aca aaa aag aaa cca tta aca caa gag cag ctt gag 1259
Leu Gln Met Ser Thr Lys Lys Lys Pro Leu Thr Gln Glu Gln Leu Glu
    385                 390                 395

gac gca cgt cgc ctt aaa gca att tat gaa aaa aag aaa aat gaa ctt 1307
Asp Ala Arg Arg Leu Lys Ala Ile Tyr Glu Lys Lys Lys Asn Glu Leu
400                 405                 410                 415

ggc tta tcc cag gaa tct gtc gca gac aag atg ggg atg ggg cag tca 1355
Gly Leu Ser Gln Glu Ser Val Ala Asp Lys Met Gly Met Gly Gln Ser
                420                 425                 430

ggc gtt ggt gct tta ttt aat ggc atc aat gca tta aat gct tat aac 1403
Gly Val Gly Ala Leu Phe Asn Gly Ile Asn Ala Leu Asn Ala Tyr Asn
            435                 440                 445

gcc gca ttg ctt gca aaa att ctc aaa gtt agc gtt gaa gaa ttt agc 1451
Ala Ala Leu Leu Ala Lys Ile Leu Lys Val Ser Val Glu Glu Phe Ser
        450                 455                 460

cct tca atc gcc aga gaa atc tac gag atg tat gaa gcg gtt agt atg 1499
Pro Ser Ile Ala Arg Glu Ile Tyr Glu Met Tyr Glu Ala Val Ser Met
    465                 470                 475

cag ccg tca ctt aga agt gag tat gag tac cct gtt ttt tct cat gtt 1547
Gln Pro Ser Leu Arg Ser Glu Tyr Glu Tyr Pro Val Phe Ser His Val
480                 485                 490                 495

cag gca ggg atg ttc tca cct gag ctt aga acc ttt acc aaa ggt gat 1595
Gln Ala Gly Met Phe Ser Pro Glu Leu Arg Thr Phe Thr Lys Gly Asp
                500                 505                 510

gcg gag aga tgg gta agc aca acc aaa aaa gcc agt gat tct gca ttc 1643
Ala Glu Arg Trp Val Ser Thr Thr Lys Lys Ala Ser Asp Ser Ala Phe
            515                 520                 525

tgg ctt gag gtt gaa ggt aat tcc atg acc gca cca aca ggc tcc aag 1691
Trp Leu Glu Val Glu Gly Asn Ser Met Thr Ala Pro Thr Gly Ser Lys
        530                 535                 540

cca agc ttt cct gac gga atg tta att ctc gtt gac cct gag cag gct 1739
Pro Ser Phe Pro Asp Gly Met Leu Ile Leu Val Asp Pro Glu Gln Ala
    545                 550                 555

gtt gag cca ggt gat ttc tgc ata gcc aga ctt ggg ggt gat gag ttt 1787
Val Glu Pro Gly Asp Phe Cys Ile Ala Arg Leu Gly Gly Asp Glu Phe
560                 565                 570                 575

acc ttc aag aaa ctg atc agg gat agc ggt cag gtg ttt tta caa cca 1835
Thr Phe Lys Lys Leu Ile Arg Asp Ser Gly Gln Val Phe Leu Gln Pro
                580                 585                 590

cta aac cca cag tac cca atg atc cca tgc aat gag agt tgt tcc gtt 1883
Leu Asn Pro Gln Tyr Pro Met Ile Pro Cys Asn Glu Ser Cys Ser Val
            595                 600                 605

gtg ggg aaa gtt atc gct agt cag tgg cct gaa gag acg ttt ggc tga     1931
Val Gly Lys Val Ile Ala Ser Gln Trp Pro Glu Glu Thr Phe Gly
        610                 615                 620

tct aga                                                             1937
Ser

<210>  2
<211>  622
<212>  PRT
<213>  Artificial sequence

<220>
<223>  Is30-ci repressor fusion protein

<400>  2

Met Arg Arg Thr Phe Thr Ala Glu Glu Lys Ala Ser Val Phe Glu Leu
1               5                   10                  15

Trp Lys Asn Gly Thr Gly Phe Ser Glu Ile Ala Asn Ile Leu Gly Ser
            20                  25                  30

Lys Pro Gly Thr Ile Phe Thr Met Leu Arg Asp Thr Gly Gly Ile Lys
        35                  40                  45

Pro His Glu Arg Lys Arg Ala Val Ala His Leu Thr Leu Ser Glu Arg
    50                  55                  60

Glu Glu Ile Arg Ala Gly Leu Ser Ala Lys Met Ser Ile Arg Ala Ile
65                  70                  75                  80

Ala Thr Ala Leu Asn Arg Ser Pro Ser Thr Ile Ser Arg Glu Val Gln
                85                  90                  95

Arg Asn Arg Gly Arg Arg Tyr Tyr Lys Ala Val Asp Ala Asn Asn Arg
            100                 105                 110

Ala Asn Arg Met Ala Lys Arg Pro Lys Pro Cys Leu Leu Asp Gln Asn
        115                 120                 125

Leu Pro Leu Arg Lys Leu Val Leu Glu Lys Leu Glu Met Lys Trp Ser
    130                 135                 140

Pro Glu Gln Ile Ser Gly Trp Leu Arg Arg Thr Lys Pro Arg Gln Lys
145                 150                 155                 160

Thr Leu Arg Ile Ser Pro Glu Thr Ile Tyr Lys Thr Leu Tyr Phe Arg
                165                 170                 175

Ser Arg Glu Ala Leu His His Leu Asn Ile Gln His Leu Arg Arg Ser
            180                 185                 190

His Ser Leu Arg His Gly Arg Arg His Thr Arg Lys Gly Glu Arg Gly
        195                 200                 205

Thr Ile Asn Ile Val Asn Gly Thr Pro Ile His Glu Arg Ser Arg Asn
    210                 215                 220

Ile Asp Asn Arg Arg Ser Leu Gly His Trp Glu Gly Asp Leu Val Ser
225                 230                 235                 240

Gly Thr Lys Asn Ser His Ile Ala Thr Leu Val Asp Arg Lys Ser Arg
                245                 250                 255

Tyr Thr Ile Ile Leu Arg Leu Arg Gly Lys Asp Ser Val Ser Val Asn
            260                 265                 270

Gln Ala Leu Thr Asp Lys Phe Leu Ser Leu Pro Ser Glu Leu Arg Lys
        275                 280                 285

Ser Leu Thr Trp Asp Arg Gly Met Glu Leu Ala Arg His Leu Glu Phe
    290                 295                 300

Thr Val Ser Thr Gly Val Lys Val Tyr Phe Cys Asp Pro Gln Ser Pro
305                 310                 315                 320

Trp Gln Arg Gly Thr Asn Glu Asn Thr Asn Gly Leu Ile Arg Gln Tyr
                325                 330                 335

Phe Pro Lys Lys Thr Cys Leu Ala Gln Tyr Thr Gln His Glu Leu Asp
            340                 345                 350

Leu Val Ala Ala Gln Leu Asn Asn Arg Pro Arg Lys Thr Leu Lys Phe
        355                 360                 365

Lys Thr Pro Lys Glu Ile Ile Glu Arg Gly Val Ala Leu Thr Asp Leu
    370                 375                 380

Gln Met Ser Thr Lys Lys Lys Pro Leu Thr Gln Glu Gln Leu Glu Asp
385                 390                 395                 400

Ala Arg Arg Leu Lys Ala Ile Tyr Glu Lys Lys Lys Asn Glu Leu Gly
                405                 410                 415

Leu Ser Gln Glu Ser Val Ala Asp Lys Met Gly Met Gly Gln Ser Gly
            420                 425                 430

Val Gly Ala Leu Phe Asn Gly Ile Asn Ala Leu Asn Ala Tyr Asn Ala
        435                 440                 445

Ala Leu Leu Ala Lys Ile Leu Lys Val Ser Val Glu Glu Phe Ser Pro
    450                 455                 460

Ser Ile Ala Arg Glu Ile Tyr Glu Met Tyr Glu Ala Val Ser Met Gln
465                 470                 475                 480

Pro Ser Leu Arg Ser Glu Tyr Glu Tyr Pro Val Phe Ser His Val Gln
                485                 490                 495

Ala Gly Met Phe Ser Pro Glu Leu Arg Thr Phe Thr Lys Gly Asp Ala
            500                 505                 510

Glu Arg Trp Val Ser Thr Thr Lys Lys Ala Ser Asp Ser Ala Phe Trp
        515                 520                 525

Leu Glu Val Glu Gly Asn Ser Met Thr Ala Pro Thr Gly Ser Lys Pro
    530                 535                 540

Ser Phe Pro Asp Gly Met Leu Ile Leu Val Asp Pro Glu Gln Ala Val
545                 550                 555                 560

Glu Pro Gly Asp Phe Cys Ile Ala Arg Leu Gly Gly Asp Glu Phe Thr
                565                 570                 575

Phe Lys Lys Leu Ile Arg Asp Ser Gly Gln Val Phe Leu Gln Pro Leu
            580                 585                 590

Asn Pro Gln Tyr Pro Met Ile Pro Cys Asn Glu Ser Cys Ser Val Val
        595                 600                 605

Gly Lys Val Ile Ala Ser Gln Trp Pro Glu Glu Thr Phe Gly
    610                 615                 620

<210>  3
<211>  1650
<212>  DNA
<213>  Artificial sequence

<220>
<221>  CDS
<222>  (1)..(1650)

<223>  IS30-gli

<400>  3
atg aga cga aca ttt aca gca gag gaa aaa gcc tct gtt ttt gaa cta      48
Met Arg Arg Thr Phe Thr Ala Glu Glu Lys Ala Ser Val Phe Glu Leu
1               5                   10                  15

tgg aag aac gga aca ggc ttc agt gaa ata gcg aat atc ctg ggt tca      96
Trp Lys Asn Gly Thr Gly Phe Ser Glu Ile Ala Asn Ile Leu Gly Ser
            20                  25                  30

aaa ccc gga acg atc ttc act atg tta agg gat act ggc ggc ata aaa      144
Lys Pro Gly Thr Ile Phe Thr Met Leu Arg Asp Thr Gly Gly Ile Lys
        35                  40                  45

ccc cat gag cgt aag cgg gct gta gct cac ctg aca ctg tct gag cgc      192
Pro His Glu Arg Lys Arg Ala Val Ala His Leu Thr Leu Ser Glu Arg
    50                  55                  60

gag gag ata cga gct ggt ttg tca gcc aaa atg agc att cgt gcg ata      240
Glu Glu Ile Arg Ala Gly Leu Ser Ala Lys Met Ser Ile Arg Ala Ile
65                  70                  75                  80

gct act gcg ctg aat cgc agt cct tcg acg atc tca cgt gaa gtt cag      288
Ala Thr Ala Leu Asn Arg Ser Pro Ser Thr Ile Ser Arg Glu Val Gln
                85                  90                  95

cgt aat cgg ggc aga cgc tat tac aaa gct gtt gat gct aat aac cga      336
Arg Asn Arg Gly Arg Arg Tyr Tyr Lys Ala Val Asp Ala Asn Asn Arg
            100                 105                 110

gcc aac aga atg gcg aaa agg cca aaa ccg tgc tta ctg gat caa aat      384
Ala Asn Arg Met Ala Lys Arg Pro Lys Pro Cys Leu Leu Asp Gln Asn
        115                 120                 125

tta cca ttg cga aag ctt gtt ctg gaa aag ctg gag atg aaa tgg tct      432
Leu Pro Leu Arg Lys Leu Val Leu Glu Lys Leu Glu Met Lys Trp Ser
    130                 135                 140

cca gag caa ata tca gga tgg tta agg cga aca aaa cca cgt caa aaa      480
Pro Glu Gln Ile Ser Gly Trp Leu Arg Arg Thr Lys Pro Arg Gln Lys
145                 150                 155                 160

acg ctg cga ata tca cct gag aca att tat aaa acg ctg tac ttt cgt      528
Thr Leu Arg Ile Ser Pro Glu Thr Ile Tyr Lys Thr Leu Tyr Phe Arg
                165                 170                 175

agc cgt gaa gcg cta cac cac ctg aat ata cag cat ctg cga cgg tcg      576
Ser Arg Glu Ala Leu His His Leu Asn Ile Gln His Leu Arg Arg Ser
            180                 185                 190

cat agc ctt cgc cat ggc agg cgt cat acc cgc aaa ggc gaa aga ggt      624
His Ser Leu Arg His Gly Arg Arg His Thr Arg Lys Gly Glu Arg Gly
        195                 200                 205

acg att aac ata gtg aac gga aca cca att cac gaa cgt tcc cga aat      672
Thr Ile Asn Ile Val Asn Gly Thr Pro Ile His Glu Arg Ser Arg Asn
    210                 215                 220

atc gat aac aga cgc tct cta ggg cat tgg gag ggc gat tta gtc tca      720

Ile Asp Asn Arg Arg Ser Leu Gly His Trp Glu Gly Asp Leu Val Ser
225                 230                 235                 240

ggt aca aaa aac tct cat ata gcc aca ctt gta gac cga aaa tca cgt      768
Gly Thr Lys Asn Ser His Ile Ala Thr Leu Val Asp Arg Lys Ser Arg
                245                 250                 255

tat acg atc atc ctt aga ctc agg ggc aaa gat tct gtc tca gta aat      816
Tyr Thr Ile Ile Leu Arg Leu Arg Gly Lys Asp Ser Val Ser Val Asn
            260                 265                 270

cag gct ctt acc gac aaa ttc ctg agt tta ccg tca gaa ctc aga aaa      864
Gln Ala Leu Thr Asp Lys Phe Leu Ser Leu Pro Ser Glu Leu Arg Lys
        275                 280                 285

tca ctg aca tgg gac aga gga atg gaa ctg gcc aga cat cta gaa ttt      912
Ser Leu Thr Trp Asp Arg Gly Met Glu Leu Ala Arg His Leu Glu Phe
    290                 295                 300

act gtc agc acc ggc gtt aaa gtt tac ttc tgc gat cct cag agt cct      960
Thr Val Ser Thr Gly Val Lys Val Tyr Phe Cys Asp Pro Gln Ser Pro
305                 310                 315                 320

tgg cag cgg gga aca aat gag aac aca aat ggg cta att cgg cag tac     1008
Trp Gln Arg Gly Thr Asn Glu Asn Thr Asn Gly Leu Ile Arg Gln Tyr
                325                 330                 335

ttt cct aaa aag aca tgt ctt gcc caa tat act caa cat gaa cta gat     1056
Phe Pro Lys Lys Thr Cys Leu Ala Gln Tyr Thr Gln His Glu Leu Asp
            340                 345                 350

ctg gtt gct gct cag cta aac aac aga ccg aga aag aca ctg aag ttc     1104
Leu Val Ala Ala Gln Leu Asn Asn Arg Pro Arg Lys Thr Leu Lys Phe
        355                 360                 365

aaa aca ccg aaa gag ata att gaa agg ggt gtt gca ttg aca gat gaa     1152
Lys Thr Pro Lys Glu Ile Ile Glu Arg Gly Val Ala Leu Thr Asp Glu
    370                 375                 380

ttc gaa tct atg tat gaa act gac tgc cgt tgg gat ggc tgc agc cag     1200
Phe Glu Ser Met Tyr Glu Thr Asp Cys Arg Trp Asp Gly Cys Ser Gln
385                 390                 395                 400

gaa ttt gac tcc caa gag cag ctg gtg cac cac atc aac agc gag cac     1248
Glu Phe Asp Ser Gln Glu Gln Leu Val His His Ile Asn Ser Glu His
                405                 410                 415

atc cac ggg gag cgg aag gag ttc gtg tgc cac tgg ggg ggc tgc tcc     1296
Ile His Gly Glu Arg Lys Glu Phe Val Cys His Trp Gly Gly Cys Ser
            420                 425                 430

agg gag ctg agg ccc ttc aaa gcc cag tac atg ctg gtg gtt cac atg     1344
Arg Glu Leu Arg Pro Phe Lys Ala Gln Tyr Met Leu Val Val His Met
        435                 440                 445

cgc aga cac act ggc gag aag cca cac aag tgc acg ttt gaa ggg tgc     1392
Arg Arg His Thr Gly Glu Lys Pro His Lys Cys Thr Phe Glu Gly Cys
    450                 455                 460

cgg aag tca tac tca cgc ctc gaa aac ctg aag acg cac ctg cgg tca     1440
Arg Lys Ser Tyr Ser Arg Leu Glu Asn Leu Lys Thr His Leu Arg Ser

465                 470                 475                 480

cac acg ggt gag aag cca tac atg tgt gag cac gag ggc tgc agt aaa      1488
His Thr Gly Glu Lys Pro Tyr Met Cys Glu His Glu Gly Cys Ser Lys
                485                 490                 495

gcc ttc agc aat gcc agt gac cga gcc aag cac cag aat cgg acc cat      1536
Ala Phe Ser Asn Ala Ser Asp Arg Ala Lys His Gln Asn Arg Thr His
            500                 505                 510

tcc aat gag aag ccg tat gta tgt aag ctc cct ggc tgc acc aaa cgc      1584
Ser Asn Glu Lys Pro Tyr Val Cys Lys Leu Pro Gly Cys Thr Lys Arg
        515                 520                 525

tat aca gat cct agc tcg ctg cga aaa cat gtc aag aca gtg cat ggt      1632
Tyr Thr Asp Pro Ser Ser Leu Arg Lys His Val Lys Thr Val His Gly
    530                 535                 540

cct gac gcc act agt taa                                              1650
Pro Asp Ala Thr Ser
545


<210>  4
<211>  549
<212>  PRT
<213>  Artificial sequence

<400>  4

Met Arg Arg Thr Phe Thr Ala Glu Glu Lys Ala Ser Val Phe Glu Leu
1               5                   10                  15


Trp Lys Asn Gly Thr Gly Phe Ser Glu Ile Ala Asn Ile Leu Gly Ser
            20                  25                  30


Lys Pro Gly Thr Ile Phe Thr Met Leu Arg Asp Thr Gly Gly Ile Lys
        35                  40                  45


Pro His Glu Arg Lys Arg Ala Val Ala His Leu Thr Leu Ser Glu Arg
    50                  55                  60


Glu Glu Ile Arg Ala Gly Leu Ser Ala Lys Met Ser Ile Arg Ala Ile
65                  70                  75                  80


Ala Thr Ala Leu Asn Arg Ser Pro Ser Thr Ile Ser Arg Glu Val Gln
                85                  90                  95


Arg Asn Arg Gly Arg Arg Tyr Tyr Lys Ala Val Asp Ala Asn Asn Arg
            100                 105                 110


Ala Asn Arg Met Ala Lys Arg Pro Lys Pro Cys Leu Leu Asp Gln Asn
        115                 120                 125

Leu Pro Leu Arg Lys Leu Val Leu Glu Lys Leu Glu Met Lys Trp Ser
    130                 135                 140

Pro Glu Gln Ile Ser Gly Trp Leu Arg Arg Thr Lys Pro Arg Gln Lys
145                 150                 155                 160

Thr Leu Arg Ile Ser Pro Glu Thr Ile Tyr Lys Thr Leu Tyr Phe Arg
                165                 170                 175

Ser Arg Glu Ala Leu His His Leu Asn Ile Gln His Leu Arg Arg Ser
            180                 185                 190

His Ser Leu Arg His Gly Arg Arg His Thr Arg Lys Gly Glu Arg Gly
        195                 200                 205

Thr Ile Asn Ile Val Asn Gly Thr Pro Ile His Glu Arg Ser Arg Asn
    210                 215                 220

Ile Asp Asn Arg Arg Ser Leu Gly His Trp Glu Gly Asp Leu Val Ser
225                 230                 235                 240

Gly Thr Lys Asn Ser His Ile Ala Thr Leu Val Asp Arg Lys Ser Arg
                245                 250                 255

Tyr Thr Ile Ile Leu Arg Leu Arg Gly Lys Asp Ser Val Ser Val Asn
            260                 265                 270

Gln Ala Leu Thr Asp Lys Phe Leu Ser Leu Pro Ser Glu Leu Arg Lys
        275                 280                 285

Ser Leu Thr Trp Asp Arg Gly Met Glu Leu Ala Arg His Leu Glu Phe
    290                 295                 300

Thr Val Ser Thr Gly Val Lys Val Tyr Phe Cys Asp Pro Gln Ser Pro
305                 310                 315                 320

Trp Gln Arg Gly Thr Asn Glu Asn Thr Asn Gly Leu Ile Arg Gln Tyr
                325                 330                 335

Phe Pro Lys Lys Thr Cys Leu Ala Gln Tyr Thr Gln His Glu Leu Asp
            340                 345                 350

Leu Val Ala Ala Gln Leu Asn Asn Arg Pro Arg Lys Thr Leu Lys Phe
        355                 360                 365

Lys Thr Pro Lys Glu Ile Ile Glu Arg Gly Val Ala Leu Thr Asp Glu
    370                 375                 380

Phe Glu Ser Met Tyr Glu Thr Asp Cys Arg Trp Asp Gly Cys Ser Gln
385                 390                 395                 400

Glu Phe Asp Ser Gln Glu Gln Leu Val His His Ile Asn Ser Glu His
                405                 410                 415

Ile His Gly Glu Arg Lys Glu Phe Val Cys His Trp Gly Gly Cys Ser
            420                 425                 430

Arg Glu Leu Arg Pro Phe Lys Ala Gln Tyr Met Leu Val Val His Met
        435                 440                 445

Arg Arg His Thr Gly Glu Lys Pro His Lys Cys Thr Phe Glu Gly Cys
    450                 455                 460

Arg Lys Ser Tyr Ser Arg Leu Glu Asn Leu Lys Thr His Leu Arg Ser
465                 470                 475                 480

His Thr Gly Glu Lys Pro Tyr Met Cys Glu His Glu Gly Cys Ser Lys
                485                 490                 495

Ala Phe Ser Asn Ala Ser Asp Arg Ala Lys His Gln Asn Arg Thr His
            500                 505                 510

Ser Asn Glu Lys Pro Tyr Val Cys Lys Leu Pro Gly Cys Thr Lys Arg
        515                 520                 525

Tyr Thr Asp Pro Ser Ser Leu Arg Lys His Val Lys Thr Val His Gly
    530                 535                 540

Pro Asp Ala Thr Ser
545

<210>  5
<211>  1785
<212>  DNA
<213>  Artificial sequence

<220>
<223>  IS-tet fusion protein

<220>
<221>  CDS
<222>  (1)..(1785)

<400> 5
atg aga cga aca ttt aca gca gag gaa aaa gcc tct gtt ttt gaa cta 48
Met Arg Arg Thr Phe Thr Ala Glu Glu Lys Ala Ser Val Phe Glu Leu
1               5                   10                  15

tgg aag aac gga aca ggc ttc agt gaa ata gcg aat atc ctg ggt tca 96
Trp Lys Asn Gly Thr Gly Phe Ser Glu Ile Ala Asn Ile Leu Gly Ser
            20                  25                  30

aaa ccc gga acg atc ttc act atg tta agg gat act ggc ggc ata aaa 144
Lys Pro Gly Thr Ile Phe Thr Met Leu Arg Asp Thr Gly Gly Ile Lys
        35                  40                  45

ccc cat gag cgt aag cgg gct gta gct cac ctg aca ctg tct gag cgc 192
Pro His Glu Arg Lys Arg Ala Val Ala His Leu Thr Leu Ser Glu Arg
    50                  55                  60

gag gag ata cga gct ggt ttg tca gcc aaa atg agc att cgt gcg ata 240
Glu Glu Ile Arg Ala Gly Leu Ser Ala Lys Met Ser Ile Arg Ala Ile
65                  70                  75                  80

gct act gcg ctg aat cgc agt cct tcg acg atc tca cgt gaa gtt cag 288
Ala Thr Ala Leu Asn Arg Ser Pro Ser Thr Ile Ser Arg Glu Val Gln
                85                  90                  95

cgt aat cgg ggc aga cgc tat tac aaa gct gtt gat gct aat aac cga 336
Arg Asn Arg Gly Arg Arg Tyr Tyr Lys Ala Val Asp Ala Asn Asn Arg
            100                 105                 110

gcc aac aga atg gcg aaa agg cca aaa ccg tgc tta ctg gat caa aat 384
Ala Asn Arg Met Ala Lys Arg Pro Lys Pro Cys Leu Leu Asp Gln Asn
        115                 120                 125

tta cca ttg cga aag ctt gtt ctg gaa aag ctg gag atg aaa tgg tct 432
Leu Pro Leu Arg Lys Leu Val Leu Glu Lys Leu Glu Met Lys Trp Ser
    130                 135                 140

cca gag caa ata tca gga tgg tta agg cga aca aaa cca cgt caa aaa 480
Pro Glu Gln Ile Ser Gly Trp Leu Arg Arg Thr Lys Pro Arg Gln Lys
145                 150                 155                 160

acg ctg cga ata tca cct gag aca att tat aaa acg ctg tac ttt cgt 528
Thr Leu Arg Ile Ser Pro Glu Thr Ile Tyr Lys Thr Leu Tyr Phe Arg
                165                 170                 175

agc cgt gaa gcg cta cac cac ctg aat ata cag cat ctg cga cgg tcg 576
Ser Arg Glu Ala Leu His His Leu Asn Ile Gln His Leu Arg Arg Ser
            180                 185                 190

cat agc ctt cgc cat ggc agg cgt cat acc cgc aaa ggc gaa aga ggt 624
His Ser Leu Arg His Gly Arg Arg His Thr Arg Lys Gly Glu Arg Gly
        195                 200                 205

acg att aac ata gtg aac gga aca cca att cac gaa cgt tcc cga aat 672
Thr Ile Asn Ile Val Asn Gly Thr Pro Ile His Glu Arg Ser Arg Asn
    210                 215                 220

atc gat aac aga cgc tct cta ggg cat tgg gag ggc gat tta gtc tca 720
Ile Asp Asn Arg Arg Ser Leu Gly His Trp Glu Gly Asp Leu Val Ser
225                 230                 235                 240

ggt aca aaa aac tct cat ata gcc aca ctt gta gac cga aaa tca cgt 768
Gly Thr Lys Asn Ser His Ile Ala Thr Leu Val Asp Arg Lys Ser Arg
                245                 250                 255

tat acg atc atc ctt aga ctc agg ggc aaa gat tct gtc tca gta aat 816
Tyr Thr Ile Ile Leu Arg Leu Arg Gly Lys Asp Ser Val Ser Val Asn
            260                 265                 270

cag gct ctt acc gac aaa ttc ctg agt tta ccg tca gaa ctc aga aaa 864
Gln Ala Leu Thr Asp Lys Phe Leu Ser Leu Pro Ser Glu Leu Arg Lys
        275                 280                 285

tca ctg aca tgg gac aga gga atg gaa ctg gcc aga cat cta gaa ttt 912
Ser Leu Thr Trp Asp Arg Gly Met Glu Leu Ala Arg His Leu Glu Phe
    290                 295                 300

act gtc agc acc ggc gtt aaa gtt tac ttc tgc gat cct cag agt cct 960
Thr Val Ser Thr Gly Val Lys Val Tyr Phe Cys Asp Pro Gln Ser Pro
305                 310                 315                 320

tgg cag cgg gga aca aat gag aac aca aat ggg cta att cgg cag tac 1008
Trp Gln Arg Gly Thr Asn Glu Asn Thr Asn Gly Leu Ile Arg Gln Tyr
                325                 330                 335

ttt cct aaa aag aca tgt ctt gcc caa tat act caa cat gaa cta gat 1056
Phe Pro Lys Lys Thr Cys Leu Ala Gln Tyr Thr Gln His Glu Leu Asp
            340                 345                 350

ctg gtt gct gct cag cta aac aac aga ccg aga aag aca ctg aag ttc 1104
Leu Val Ala Ala Gln Leu Asn Asn Arg Pro Arg Lys Thr Leu Lys Phe
        355                 360                 365

aaa aca ccg aaa gag ata att gaa agg ggt gtt gca ttg aca gat gaa 1152
Lys Thr Pro Lys Glu Ile Ile Glu Arg Gly Val Ala Leu Thr Asp Glu
    370                 375                 380

ttc agg tct aga tta gat aaa agt aaa gtg att aac agc gca tta gag 1200
Phe Arg Ser Arg Leu Asp Lys Ser Lys Val Ile Asn Ser Ala Leu Glu
385                 390                 395                 400

ctg ctt aat gag gtc gga atc gaa ggt tta aca acc cgt aaa ctc gcc 1248
Leu Leu Asn Glu Val Gly Ile Glu Gly Leu Thr Thr Arg Lys Leu Ala
                405                 410                 415

cag aag ctt ggt gta gag cag cct aca ctg tat tgg cat gta aaa aat 1296
Gln Lys Leu Gly Val Glu Gln Pro Thr Leu Tyr Trp His Val Lys Asn
            420                 425                 430

aag cgg gct ttg ctc gac gcc tta gcc att gag atg tta gat agg cac 1344
Lys Arg Ala Leu Leu Asp Ala Leu Ala Ile Glu Met Leu Asp Arg His
        435                 440                 445

cat act cac ttt tgc cct tta aaa ggg gaa agc tgg caa gat ttt tta 1392
His Thr His Phe Cys Pro Leu Lys Gly Glu Ser Trp Gln Asp Phe Leu
    450                 455                 460

cgc aat aac gct aaa agt ttt aga tgt gct tta cta agt cat cgc aat 1440
Arg Asn Asn Ala Lys Ser Phe Arg Cys Ala Leu Leu Ser His Arg Asn
465                 470                 475                 480

gga gca aaa gta cat tca gat aca cgg cct aca gaa aaa cag tat gaa 1488

Gly Ala Lys Val His Ser Asp Thr Arg Pro Thr Glu Lys Gln Tyr Glu
                485                 490                 495

act ctc gaa aat caa tta gcc ttt tta tgc caa caa ggt ttt tca cta      1536
Thr Leu Glu Asn Gln Leu Ala Phe Leu Cys Gln Gln Gly Phe Ser Leu
            500                 505                 510

gag aac gcg tta tat gca ctc agc gct gtg ggg cat ttt act tta ggt      1584
Glu Asn Ala Leu Tyr Ala Leu Ser Ala Val Gly His Phe Thr Leu Gly
        515                 520                 525

tgc gta ttg gaa gat caa gag cat caa gtc gct aaa gaa gaa agg gaa      1632
Cys Val Leu Glu Asp Gln Glu His Gln Val Ala Lys Glu Glu Arg Glu
    530                 535                 540

aca cct act act gat agt atg ccg cca tta tta cga caa gct atc gaa      1680
Thr Pro Thr Thr Asp Ser Met Pro Pro Leu Leu Arg Gln Ala Ile Glu
545                 550                 555                 560

tta ttt gat cac caa ggt gca gag cca gcc ttc tta ttc ggc ctt gaa      1728
Leu Phe Asp His Gln Gly Ala Glu Pro Ala Phe Leu Phe Gly Leu Glu
                565                 570                 575

ttg atc ata tgc gga tta gaa aaa caa ctt aaa tgt gaa agt ggg tct      1776
Leu Ile Ile Cys Gly Leu Glu Lys Gln Leu Lys Cys Glu Ser Gly Ser
            580                 585                 590

act agt taa                                                          1785
Thr Ser

<210>  6
<211>  594
<212>  PRT
<213>  Artificial sequence

<220>
<223>  IS-tet fusion protein

<400>  6

Met Arg Arg Thr Phe Thr Ala Glu Glu Lys Ala Ser Val Phe Glu Leu
1               5                   10                  15

Trp Lys Asn Gly Thr Gly Phe Ser Glu Ile Ala Asn Ile Leu Gly Ser
            20                  25                  30

Lys Pro Gly Thr Ile Phe Thr Met Leu Arg Asp Thr Gly Gly Ile Lys
        35                  40                  45

Pro His Glu Arg Lys Arg Ala Val Ala His Leu Thr Leu Ser Glu Arg
    50                  55                  60

Glu Glu Ile Arg Ala Gly Leu Ser Ala Lys Met Ser Ile Arg Ala Ile
65                  70                  75                  80

Ala Thr Ala Leu Asn Arg Ser Pro Ser Thr Ile Ser Arg Glu Val Gln
                85                  90                  95

Arg Asn Arg Gly Arg Arg Tyr Tyr Lys Ala Val Asp Ala Asn Asn Arg
            100                 105                 110

Ala Asn Arg Met Ala Lys Arg Pro Lys Pro Cys Leu Leu Asp Gln Asn
        115                 120                 125

Leu Pro Leu Arg Lys Leu Val Leu Glu Lys Leu Glu Met Lys Trp Ser
    130                 135                 140

Pro Glu Gln Ile Ser Gly Trp Leu Arg Arg Thr Lys Pro Arg Gln Lys
145                 150                 155                 160

Thr Leu Arg Ile Ser Pro Glu Thr Ile Tyr Lys Thr Leu Tyr Phe Arg
                165                 170                 175

Ser Arg Glu Ala Leu His His Leu Asn Ile Gln His Leu Arg Arg Ser
            180                 185                 190

His Ser Leu Arg His Gly Arg Arg His Thr Arg Lys Gly Glu Arg Gly
        195                 200                 205

Thr Ile Asn Ile Val Asn Gly Thr Pro Ile His Glu Arg Ser Arg Asn
    210                 215                 220

Ile Asp Asn Arg Arg Ser Leu Gly His Trp Glu Gly Asp Leu Val Ser
225                 230                 235                 240

Gly Thr Lys Asn Ser His Ile Ala Thr Leu Val Asp Arg Lys Ser Arg
                245                 250                 255

Tyr Thr Ile Ile Leu Arg Leu Arg Gly Lys Asp Ser Val Ser Val Asn
            260                 265                 270

Gln Ala Leu Thr Asp Lys Phe Leu Ser Leu Pro Ser Glu Leu Arg Lys
        275                 280                 285

Ser Leu Thr Trp Asp Arg Gly Met Glu Leu Ala Arg His Leu Glu Phe
    290                 295                 300

Thr Val Ser Thr Gly Val Lys Val Tyr Phe Cys Asp Pro Gln Ser Pro
305                 310                 315                 320

Trp Gln Arg Gly Thr Asn Glu Asn Thr Asn Gly Leu Ile Arg Gln Tyr
                325                 330                 335

Phe Pro Lys Lys Thr Cys Leu Ala Gln Tyr Thr Gln His Glu Leu Asp
            340                 345                 350

Leu Val Ala Ala Gln Leu Asn Asn Arg Pro Arg Lys Thr Leu Lys Phe
        355                 360                 365

Lys Thr Pro Lys Glu Ile Ile Glu Arg Gly Val Ala Leu Thr Asp Glu
    370                 375                 380

Phe Arg Ser Arg Leu Asp Lys Ser Lys Val Ile Asn Ser Ala Leu Glu
385                 390                 395                 400

Leu Leu Asn Glu Val Gly Ile Glu Gly Leu Thr Thr Arg Lys Leu Ala
                405                 410                 415

Gln Lys Leu Gly Val Glu Gln Pro Thr Leu Tyr Trp His Val Lys Asn
            420                 425                 430

Lys Arg Ala Leu Leu Asp Ala Leu Ala Ile Glu Met Leu Asp Arg His
        435                 440                 445

His Thr His Phe Cys Pro Leu Lys Gly Glu Ser Trp Gln Asp Phe Leu
    450                 455                 460

Arg Asn Asn Ala Lys Ser Phe Arg Cys Ala Leu Leu Ser His Arg Asn
465                 470                 475                 480

Gly Ala Lys Val His Ser Asp Thr Arg Pro Thr Glu Lys Gln Tyr Glu
                485                 490                 495

Thr Leu Glu Asn Gln Leu Ala Phe Leu Cys Gln Gln Gly Phe Ser Leu
            500                 505                 510

Glu Asn Ala Leu Tyr Ala Leu Ser Ala Val Gly His Phe Thr Leu Gly
        515                 520                 525

Cys Val Leu Glu Asp Gln Glu His Gln Val Ala Lys Glu Glu Arg Glu
    530                 535                 540

Thr Pro Thr Thr Asp Ser Met Pro Pro Leu Leu Arg Gln Ala Ile Glu
545                 550                 555                 560

Leu Phe Asp His Gln Gly Ala Glu Pro Ala Phe Leu Phe Gly Leu Glu

                565                 570                 575
Leu Ile Ile Cys Gly Leu Glu Lys Gln Leu Lys Cys Glu Ser Gly Ser
            580                 585                 590
Thr Ser